# EUROPEAN PATENT APPLICATION

(11) **EP 1 862 475 A1**
(43) Date of publication of application: **05.12.2007**
(21) Application number: 07110666.0
(22) Date of filing: 25.01.2002
(51) Int. Cl.: C07K 14/475, C12N 15/12, A61K 47/48, A61K 38/18

(54) **Polymer conjugates of neublastin and methods of using same**

(30) Priority: 01.02.2001 US 266071 P
(62) Divisional of application: 02714792.5
(71) Applicant: Biogen Idec MA Inc., Cambridge, MA 02142 (US)
(72) Inventor: Sah, Dinah,W., Y., Boston, MA 02114 (US); Pepinsky, Blake, R., Arlington, MA 02474 (US); Borjack-Sjodin, Paula, Ann, Waltham, MA 02453 (US); Miller, Stephan, S., Arlington, MA 02474 (US); Rossomando, Anthony, Revere, MA 02151 (US)
(74) Representative: Pohlman, Sandra M.

(57) **Abstract**

A variant neublastin polypeptide suitable for formulation of a conjugate comprising the variant neublastin polypeptide coupled to a polymer containing a polymer containing a polyalkylene glycol moiety is disclosed. The present conjugate has prolonged bioavailability and, in preferred embodiments, prolonged biological activity relative to non-modified or wild-type forms of neublastin. The conjugates of the invention are usefully employed in therapeutic as well as non-therapeutic, e.g., diagnostic, applications.

## Description

### FIELD OF THE INVENTION

The invention relates generally to polypeptides and more particularly to modified neurotrophic polypeptides and methods of using these modified polypeptides.

### BACKGROUND OF THE INVENTION

Neurotrophic factors are naturally-occurring proteins that promote survival, maintain phenotypic differentiation, prevent degeneration, and enhance the activity of neuronal cells and tissues. Neurotrophic factors are isolated from neural tissue and from non-neural tissue that is innervated by the nervous system, and have been classified into functionally and structurally related groups, also referred to as families, superfamilies, or subfamilies. Among the neurotrophic factor superfamilies are the fibroblast growth factor, neurotrophin, and transforming growth factor-β (TGF-β) superfamilies. Individual species of neurotrophic factors are distinguished by their physical structure, their interaction with their cognate receptors, and their affects on various types of nerve cells. Classified within the TGF-β superfamily are the glial cell line-derived neurotrophic factor (GDNF) ligands, which include GDNF, persephin (PSP) and neurturin (NTN).

The ligands of the GDNF subfamily have in common their ability to induce signaling through the RET receptor tyrosine kinase. These three ligands of the GDNF subfamily differ in their relative affinities for a family of neurotrophic receptors, the GFRα receptors.

A recently described neurotrophic factor is "neublastin," or "NBN." Neublastin is classified within the GDNF subfamily because it shares regions of homology with other GDNF ligands and because of its ability to bind to, and activate, RET. Unlike other GDNF ligands, neublastin is highly selective for the GFRα3-RET receptor complex. In addition, NBN contains unique subregions in its amino acid sequence.

Unfortunately, neublastin is cleared rapidly by the body. This rapid clearance can frustrate the use of neublastin in therapeutic applications. Thus, a need exists to identify neublastin variants that have increased bioavailability.

### Summary of the Invention

The present invention is based in part on the discovery of novel forms of neublastin that show enhanced pharmokinetic and bioavailability properties *in vivo.* These novel forms include variant neublastin polypeptides conjugated to polymeric molecules.

In one aspect, the invention features a variant neublastin polypeptide, or mutein neublastin polypeptide, that includes an amino acid sequence having one or more amino acid substitutions at solvent-exposed positions of the mature neublastin dimer. The present substitutions introduce into the native neublastin polypeptide one or more sites at which substances, such as naturally occurring or synthetic polymers, can be attached to the polypeptide so as to enhance its solubility, and hence its bioavailability, in vivo. Preferably, the present variant polypeptides include an amino acids sequence at least 70% identical to amino acids 8-113 of SEQ ID NO:1. The variant neublastin polypeptide includes one or more amino acid substitutions in which an amino acid other than arginine occurs at position 14 in the amino acid sequence of the variant polypeptide, an amino acid other than arginine at position 39 occurs in the amino acid sequence of the variant polypeptide, an amino acid other than arginine at position 68 occurs in the variant polypeptide, or an amino acid other than asparagine at position 95 occurs in the variant polypeptide, when the positions of the amino acids are numbered in accordance with the polypeptide sequence of SEQ ID NO: 1.

As used herein, "wild-type neublastin" or "wt-NBN" refers to a naturally-occurring or native neublastin polypeptide sequence, such as that of rat, mouse, or human neublastin (see, e.g., SEQ ID NO: 2, 3, or 4). Specific variant neublastin polypeptides are referred to herein as "NBN-X₁N₁X₂" or "X₁N₁X₂-NBN" wherein X₁ refers to an amino acid of a wild-type neublastin polypeptide, N₁, refers to the numerical position of the X₁ amino acids in the sequence, as numbered according to SEQ ID NO: 1. X₂ refers to an amino acid substituted for the wild-type amino acid at the indicated numerical position in the sequence. Thus, for example, NBN-N95K identifies the variant neublastin polypeptides in which the asparagine at position 95 is replaced by a lysine.

The variant neublastin polypeptide can be provided as a multimeric polypeptide. For example, the variant neublastin polypeptide can be provided as a dimer that includes at least one variant neublastin polypeptide. In some embodiments, the dimer is a homodimer of variant neublastin polypeptides. In other embodiments, the dimer is a heterodimer that includes one variant neublastin polypeptide and one wild-type neublastin polypeptide. Other dimers may include two different variant neublastin polypeptide forms.

In some embodiments, the variant neublastin polypeptide includes amino acids 1-7 of SEQ ID NO: 1 in addition to amino acids 8-113.

In preferred embodiments, the variant neublastin polypeptide, when dimerized, binds GFRα3. In additional preferred embodiments, the variant neublastin polypeptide, when dimerized, stimulates tyrosine phosphorylation of a RET polypeptide, either on its own or when bound to GFRα3.

In still other preferred embodiments, the variant neublastin polypeptide, when dimerized, enhances neuron survival, *e.g.,* enhances survival of a sensory neuron.

In still other preferred embodiments, the variant neublastin polypeptide, when dimerized, normalizes pathological changes of a neuron, such as a sensory neuron.

In yet further preferred embodiments, the variant neublastin polypeptide, when dimerized, enhances survival of a neuron, e.g., an autonomic neuron, or a dopaminergic neuron.

In some embodiments, the variant polypeptide includes two, three, or more of the amino acid substitutions selected from the group consisting of an amino acid other than arginine at position 14 in the amino acid sequence of the variant polypeptide, an amino acid other than arginine at position 39 in the amino acid sequence of the variant polypeptide, an amino acid other than arginine at position 68 of the variant polypeptide, and an amino acid other than asparagine at position 95 of the variant polypeptide.

In preferred embodiments, the amino acid at one or more of the positions, 14, 39, 68, and 95 is lysine.

Preferably, amino acids 8-94 and 96-113 of the variant neublastin polypeptide are at least 90% identical to amino acids 8-94 and 96-113 of SEQ ID NO:1. More preferably, the amino acids sequences are at least 95% identical thereto. Most preferably, the amino acid sequence of the variant neublastin polypeptide includes the amino acid sequence of a naturally occurring rat, human or mouse neublastin polypeptide at amino acids 8-94 and 96-113 of the variant neublastin polypeptide. For example, amino acids 8-94 and 96-113 of the variant neublastin polypeptide can include the amino acid sequence of amino acids 8-94 and 96-113 of SEQ ID NO:2, SEQ ID NO:3 or SEQ ID NO: 4.

Also provided by the invention is a fusion protein or polypeptide that includes a variant neublastin polypeptide or a wild-type neublastin polypeptide, or a protein that is a dimer of two neublastin fusion proteins. Neublastin fusion proteins also have enhanced pharmacokinetic and bioavailability properties in vivo.

In another aspect, the invention provides a nucleic acid molecule encoding a variant neublastin polypeptide. The nucleic acid encoding the variant neublastin polypeptide is preferably provided in a vector, *e.g.*, an expression vector. A variant neublastin nucleic acid, or a vector including the same, can be provided in a cell. The cell can be, *e.g.,* a mammalian cell, fungal cell, yeast cell, insect cell, or bacterial cell. A preferred mammalian cell is a Chinese hamster ovary cell ("CHO cell").

Also provided by the invention is a method of making a variant neublastin polypeptide, by culturing a cell containing a nucleic acid encoding a variant neublastin nucleic acid under conditions allowing for expression of a variant neublastin polypeptide. If desired, the variant neublastin polypeptide can then be recovered. The invention further includes the variant neublastin polypeptide produced by the cell. Similar nucleic acids, vectors, host cells, and polypeptide production methods are disclosed herein for the fusion proteins (such as the neublastin-serum albumin fusion proteins) of this invention.

Also provided by the invention is a composition that includes a neublastin polypeptide or variant neublastin polypeptide coupled to a non-naturally occurring polymer. The variant neublastin polypeptide in the composition preferably includes an amino acid sequence at least 70% identical to amino acids 8-113 of SEQ ID NO:1, provided that the variant neublastin polypeptide includes one or more of the amino acid substitutions selected from the group consisting of an amino acid other than arginine at position 14 in the amino acid sequence of the variant polypeptide, an amino acid other than arginine at position 39 in the amino acid sequence of the variant polypeptide, an amino acid other than arginine at position 68 of the variant polypeptide, and an amino acid other than asparagine at position 95 of the variant polypeptide, wherein the positions of the amino acids are numbered in accordance with the polypeptide sequence of SEQ ID NO:1.

In preferred embodiments, the polymer comprises a polyalkylene glycol moiety, *e.g.,* polyethylene glycol moiety (PEG).

In preferred embodiments, the polyalkylene glycol moiety is coupled to an amine group of the neublastin polypeptide, or a lysine in a variant neublastin polypeptide.

Coupling can occur via a N-hydroxylsuccinimide (NHS) active ester. The active ester can be, *e.g.,* PEG succinimidyl succinate (SS-PEG), succinimidyl butyrate (SPB-PEG), or succinimidyl propionate (SPA-PEG).

The polyalkylene glycol moiety can be, *e.g*., carboxymethyl-NHS, norleucine-NHS, SC-PEG, tresylate, aldehyde, epoxide, carbonylimidazole, or PNP carbonate.

In some embodiments, the polyalkylene glycol moiety is coupled to a cysteine group of the neublastin polypeptide or variant neublastin polypeptide. For example, coupling can occur via a maleimide group, a vinylsulfone group, a haloacetate group, and a thiol group.

In some embodiments, the neublastin polypeptide or variant neublastin polypeptide in the composition is glycosylated. When the neublastin polypeptide or variant polypeptide is glycosylated, the polymer can be coupled to a carbohydrate moiety of the glycosylated neublastin polypeptide or variant neublastin polypeptide. For example, the polymer can be coupled to the glycosylated neublastin polypeptide or variant neublastin polypeptide following oxidation of a hydrazole group or an amino group of the glycosylated neublastin polypeptide or variant neublastin polypeptide, or oxidation of a reactive group of the polymer.

In various embodiments, the neublastin polypeptide or variant neublastin polypeptide comprises one, two, three, or four PEG moieties.

In preferred embodiments, the neublastin polypeptide, variant neublastin polypeptide or polymer conjugate has a longer serum half-life relative to the half-life of the polypeptide or variant polypeptide in the absence of the polymer.

In preferred embodiments, the neublastin polypeptide, variant neublastin polypeptide or polymer conjugate in the complex has a physiological activity selected from the group consisting of: GFRα3 binding, RET activation, normalization of pathological changes of a neuron, or enhancing neuron survival.

By "normalization of pathological changes of a neuron" is meant that the present conjugate induces a change in one or more of the following cellular parameters: expression level of a structural protein, a neurotrophic factor receptor, an ion channel, or a neurotransmitter, or, induces a change in cellular morphology, in each case so as to substantially restore such parameter to the level thereof in a neuron of the same or similar phenotype that is unaffected by disease, degeneration, insult, or injury. The normalization of pathological changes of a neuron can be monitored immunohistochemically, or by assessing changes in the levels of secreted or shed cellular products, or by assessing in vivo changes in behavior physiologically attributable to function of the affected neuron(s). For example, in the case of pathologic changes associated with a neuropathic pain syndrome, pain behaviors such as hyperalgesia, hypoalgesia, or allodynia, can be monitored.

By "enhancing neuron survival" is meant extending the survival of an affected neuron beyond the survival period observed in a corresponding neuron affected by the same type of disease, disorder, insult, or injury but not treated with the neublastin conjugate or fusion protein of this invention.

In some embodiments, the polymer is coupled to the polypeptide at a site on the neublastin that is an N terminus. In some embodiments, the polymer is coupled to the polypeptide at a site in a non-terminal amino acid of the neublastin polypeptide or variant neublastin polypeptide.

In preferred embodiments, the polymer is coupled to a solvent exposed amino acid of the neublastin polypeptide or variant neublastin polypeptide.

In preferred embodiments, the polymer is coupled to the neublastin polypeptide or variant neublastin polypeptide at a residue selected from the group consisting of the amino terminal amino acid of the variant polypeptide, position 14 in the amino acid sequence of the neublastin polypeptide or variant neublastin polypeptide, position 39 in the amino acid sequence of the neublastin polypeptide or variant neublastin polypeptide, position 68 in the amino acid sequence of the neublastin polypeptide or variant neublastin polypeptide, and position 95 in the amino acid sequence of the neublastin polypeptide or variant polypeptide.

Also provided by the invention is a pharmaceutical composition comprising a physiologically acceptable vehicle containing or having dispersed therein a neublastin polypeptide, a variant neublastin polypeptide, or a conjugate of the present invention.

In a further aspect, the invention includes a stable, aqueously soluble conjugated neublastin polypeptide or variant neublastin polypeptide complex comprising a neublastin polypeptide or variant neublastin polypeptide coupled to a polyethylene glycol moiety, wherein the neublastin polypeptide or variant neublastin polypeptide is coupled to the polyethylene glycol moiety by a labile bond. In some embodiments, the labile bond is cleavable by biochemical hydrolysis, proteolysis, or sulfhydryl cleavage. In preferred embodiments, the labile bond is cleavable under in vivo conditions.

Also provided by the invention is a method for making a modified neublastin polypeptide that has prolonged activity, in vitro or in vivo, relative to a wild-type neublastin by providing a neublastin polypeptide or variant neublastin polypeptide, and coupling the polypeptide or modified variant neublastin polypeptide to a non-naturally occurring polymer moiety, thereby forming a coupled polymer neublastin polypeptide composition.

In a further aspect, the invention provides a method of treating or preventing a nervous system disorder in a subject (such as a human), by administering to a subject in need thereof a therapeutically effective amount of a variant neublastin polypeptide, a composition containing a neublastin polypeptide or variant neublastin polypeptide coupled to a polymer, or a complex that includes a stable, aqueously soluble conjugated neublastin polypeptide or variant neublastin polypeptide complex comprising a neublastin polypeptide or variant neublastin polypeptide coupled to a polyethylene glycol moiety.

Preferably, the nervous system disorder is a peripheral nervous disorder, such as a peripheral neuropathy or a neuropathic pain syndrome. Humans are preferred subjects for treatment.

Administration can be, *e.g.*, systemic or local.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

Other features and advantages of the invention will be apparent from the following detailed description and claims.

### DETAILED DESCRIPTION OF THE INVENTION

The invention provides novel variant neublastin polypeptides that can be modified to enhance their pharmokinetic and bioavailability properties, Preferred variant neublastin polypeptides have altered amino acid sequences which facilitate coupling to a polymer agent such as a polyalkylene glycol polymer.

### Variant Neublastin Polypeptides

The invention provides neublastin polypeptides that have variant amino acid sequences with respect to a wild-type neublastin polypeptide sequence. Amino acid sequences of human and mouse neublastin polypeptides are disclosed in WO00/01815. Examples of variant neublastin polypeptides according to the invention are presented in Table 1.

Preferably, the altered residues in the variant neublastin polypeptide are chosen to facilitate coupling of a polymer such as a polyalkylene glycol polymer at the location of the modified amino acid. Preferred sites of modification of a neublastin polypeptide are those at solvent accessible regions in the neublastin polypeptide. Such sites can be chosen based on inspection of the crystal structure of the related neurotrophic factor, GDNF, whose crystal structure is described in Nat. Struct. Biol. 4:435-38, 1997. Sites can also be chosen based on the structural-functional information provided for persephin/neublastin chimeric proteins. These chimeras are described in J. Biol. Chem. 275:3412-20,2000. An exemplary listing of solvent accessible or surface exposed neublastin amino acids identified through this methodology is as set forth in Table 2.

The invention includes a variant neublastin polypeptide that includes an amino acid sequence that is at least 70% identical to amino acids 8-113 of SEQ ID NO: 1, which is shown in Table 1. In some embodiments, one or more of the arginines at position 14, position 39, position 68, or the asparagine at position 95, in the amino acid sequence of the polypeptide, is replaced by an amino acid other than arginine or asparagine. Preferably, the wild-type type amino acid is replaced with lysine or cysteine.

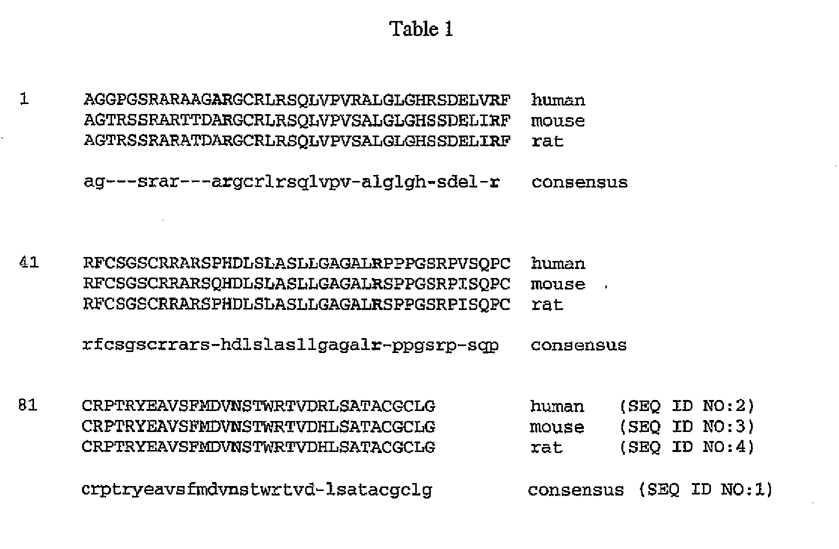

Consensus sequence:

Ala Gly Xaa1 Xaa2 Xaa3 Ser Arg Ala Arg Xaa4 Xaa5 Xaa6 Ala Arg Gly Cys

Arg Leu Arg Ser Gln Leu Val Pro Val Xaa7 Ala Leu Gly Leu Gly His Xaa8 Ser

Asp Glu Leu Xaa9 Arg Phe Arg Phe Cys Ser Gly Ser Cys Arg Arg Ala Xaa9

Ser Xaa10 His Asp Leu Ser Leu Ala Ser Leu Leu Gly Ala Gly Ala Leu Arg
Xaa11 Pro Pro Gly Ser Arg Pro Xaa12 Ser Gln Pro Cys Cys Arg Pro Thr Arg
Tyr Glu Ala Val Ser Phe Met Asp Val Lys Ser Thr Trp Arg Thr Val Asp
Xaa13 Leu Ser Ala Thr Ala Cys Gly Cys Leu Gly
wherein
Xaa1 is Gly or Thr
Xaa2 is Pro or Arg
Xaa3 is Gly or Ser
Xaa4 is Ala or Thr
Xaa5 is Ala or Thr
Xaa6 is Gly or Asp
Xaa7 is Arg or Ser
Xaa8 is Arg or Ser
Xaa9 is Val or Ile
Xaa10 is Pro or Gln
Xaa11 is Pro or Ser
Xaa12 is Val or Ile
Xaa13 is Arg or His

Table 2 provides a list of residues and numbers in human neublastin that are expected to be surface exposed. Surface exposed residues were determined by examining the structure of the rat GDNF dimer formed by chains A and B (PDB code 1AGQ) and determining whether a residue was on the surface of the structure. This structure was then compared to a sequence alignment of GDNF and neublastin in Baloh et al., Neuron, vol 21, pg 1291, 1998 to determine the proper residues in neublastin. The numbering scheme is that shown in Table 1.

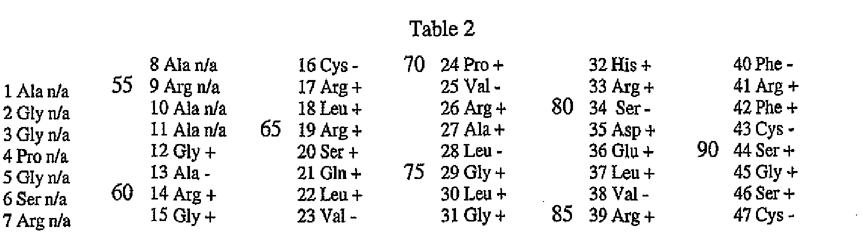

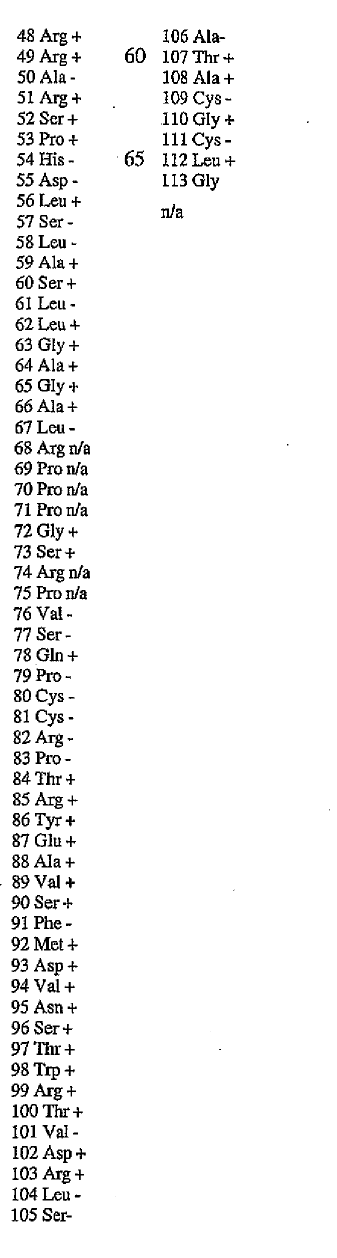

n/a indicates that the residues are not present in the structure of GDNF. This is either because of construct design, flexible regions, or inserts in neublastin relative to GDNF (residues 68-71).
- indicates the residues are buried and not on the surface or are cysteine residues involved in disulfide bonds. As this protein is a cysteine knot, a great majority of the residues are on the surface.
+ indicates that this residue is surface exposed in the GDNF structure, and therefore is presumed to be surface exposed in neublastin, although the loop containing residues 66-75 is visible in only one of the GDNF monomers (presumably flexible). This loop also contains a 4 residue insert in neublastin relative to GDNF.

As used herein, "identity" and "homologous" or "homology" are used interchangeably and refer to the sequence similarity between two polypeptides, molecules or between two nucleic acids. When a position in both of the two compared sequences is occupied by the same base or amino acid monomer subunit (for instance, if a position in each of the two DNA molecules is occupied by adenine, or a position in each of two polypeptides is occupied by a lysine), then the respective molecules are homologous at that position. The percentage homology between two sequences is a function of the number of matching or homologous positions shared by the two sequences divided by the number of positions compared x 100. For instance, if 6 of 10 of the positions in two sequences are matched or are homologous, then the two sequences are 60% homologous. By way of example, the DNA sequences CTGACT and CAGGTT share 50% homology (3 of the 6 total positions are matched). Generally, a comparison is made when two sequences are aligned to give maximum homology. Such alignment can be provided using, for instance, the method of Needleman et al., J. Mol Biol. 48: 443-453 (1970), implemented conveniently by computer programs such as the Align program (DNAstar, Inc.). "Similar" sequences are those which, when aligned, share identical and similar amino acid residues, where similar residues are conservative substitutions for, or "allowed point mutations" of, corresponding amino acid residues in an aligned reference sequence. In this regard, a "conservative substitution" of a residue in a reference sequence is a substitution by a residue that is physically or functionally similar to the corresponding reference residue, e.g., that has a similar size, shape, electric charge, chemical properties, including the ability to form covalent or hydrogen bonds, or the like. Thus, a "conservative substitution variant" sequence is one which differs from a reference sequence or a wild-type sequence in that one or more conservative substitutions or allowed point mutations are present.

In preferred embodiments, a polypeptide according to the invention is 80 %, 85%, 90%, 95%, 98% or 99% identical to amino acids 8-113 of SEQ ID NO:1. In some embodiments, the amino acid sequence of the variant neublastin polypeptide includes the amino acid sequence of a naturally occurring rat, human or mouse neublastin polypeptide at amino acids 1-94 and 96-113 of the variant neublastin polypeptide, e.g., the polypeptide has the amino acid sequence of SEQ ID NOs: 2, 3, or 4 at these positions.

A variant neublastin polypeptide differing in sequence from those disclosed in SEQ ID NOs:1-4 may include one or more conservative amino acid substitutions. Alternatively, or in addition, the variant neublastin polypeptide may differ by one or more non conservative amino acid substitutions, or by deletions or insertions. Preferably, the substitutions, insertions or deletions do not abolish the isolated protein's biological activity.

Conservative substitutions typically include the substitution of one amino acid for another with similar characteristics such as substitutions within the following groups: valine, alanine and glycine; leucine, valine, and isoleucine; aspartic acid and glutamic acid; asparagine and glutamine; serine, cysteine, and threonine; lysine and arginine; and phenylalanine and tyrosine. The non polar hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Any substitution of one member of the above-mentioned polar, basic or acidic groups by another member of the same group can be deemed a conservative substitution.

Other substitutions can be readily identified by workers of ordinary skill. For example, for the amino acid alanine, a substitution can be taken from any one of D-alanine, glycine, beta-alanine, L-cysteine and D-cysteine. For lysine, a replacement can be any one of D-lysine, arginine, D-arginine, homo-arginine, methionine, D-methionine, ornithine, or D-ornithine. Generally, substitutions in functionally important regions that may be expected to induce changes in the properties of isolated polypeptides are those in which: (i) a polar residue, e.g., serine or threonine, is substituted for (or by) a hydrophobic residue, e.g., leucine, isoleucine, phenylalanine, or alanine; (ii) a cysteine residue is substituted for (or by) any other residue; (iii) a residue having an electropositive side chain, e.g., lysine, arginine or histidine, is substituted for (or by) a residue having an electronegative side chain, e.g., glutamic acid or aspartic acid; or (iv) a residue having a bulky side chain, *e.g.,* phenylalanine, is substituted for (or by) one not having such a side chain, e.g., glycine. The likelihood that one of the foregoing non-conservative substitutions may alter functional properties of the protein is also correlated to the position of the substitution with respect to functionally important regions of the protein: some non-conservative substitutions may accordingly have little or no effect on biological properties.

Also provided by the invention are multimeric polypeptides that include a variant neublastin polypeptide. The multimeric polypeptides are preferably provided as purified multimeric polypeptides. Examples of multimeric complexes include, *e.g.,* dimeric complexes. The multimeric complex can be provided as a heteromeric or homomeric complex. Thus, the multimeric complex can be a heterodimeric complex including one variant neublastin polypeptide and one non-variant neublastin or a heterodimeric complex including two or more variant neublastin polypeptides.

In some embodiments, the variant neublastin polypeptide binds GFRα3. Preferably, binding of the variant neublastin polypeptide stimulates phosphorylation of a RET polypeptide. To determine whether a polypeptide binds GFRα3, assays can be performed as described in WO00/01815. For example, the presence of neublastin in the media of CHO cell line supernatants can be described using a modified form of a ternary complex assay described by Sanicola et al. (Proc. Natl. Acad. Sci. USA, 1997, 94: 6238). In this assay, the ability of GDNF-like molecules can be evaluated for their ability to mediate binding between the extracellular domain of RET and the various co-receptors, GFRα1, GFRα2, and GFRα3. Soluble forms of RET and the co-receptors are generated as fusion proteins. A fusion protein between the extracellular domain of rat RET and placental alkaline phosphatase (RET-AP) and a fusion protein between the extracellular domain of rat GFRα-1 (disclosed in published application WO9744356; November 27,1997, herein incorporated by reference) and the Fc domain of human IgG1 (rGFR(α1-Ig) have been described (Sanicola et al., Proc. Natl. Acad. Sci, USA 1997, 94: 6238).

In some embodiments, the variant neublastin polypeptide enhances survival of a neuron, or normalizes pathological changes of a neuron or both. Assays for determining whether a polypeptide enhances survival of a neuron, or normalizes pathological changes of a neuron, are described in, e.g., WO00/01815. Preferably, the neuron is a sensory neuron, an autonomic neuron, or a dopaminergic neuron.

### Synthesis and Isolation of Variant Neublastin Polypeptides

Variant neublastin polypeptides can be isolated using methods known in the art. Naturally occurring neublastin polypeptides can be isolated from cells or tissue sources by an appropriate purification scheme using standard protein purification techniques. Alternatively, variant neublastin polypeptides can be synthesized chemically using standard peptide synthesis techniques. The synthesis of short amino acid sequences is well established in the peptide art. See, e.g., Stewart, et al., Solid Phase Peptide Synthesis (2d ed., 1984).

In another embodiment, variant neublastin polypeptides are produced by recombinant DNA techniques. For example, a nucleic acid molecule encoding a variant neublastin polypeptide can be inserted into a vector, e.g., an expression vector, and the nucleic acid can be introduced into a cell. Suitable cells include, e.g., mammalian cells (such as human cells or Chinese hamster ovary cells), fungal cells, yeast cells, insect cells, and bacterial cells. When expressed in a recombinant cell, the cell is preferably cultured under conditions allowing for expression of a variant neublastin polypeptide. The variant neublastin polypeptide can be recovered from a cell suspension if desired. By "recovered" is meant that the variant polypeptide is removed from those components of a cell or culture medium in which it is present prior to the recovery process. The recovery process may include one or more refolding or purification steps.

Variant neublastin polypeptides can be constructed using any of several methods known in the art. One such method is site-directed mutagenesis, in which a specific nucleotide (or, if desired a small number of specific nucleotides) is changed in order to change a single amino acid (or, if desired, a small number of predetermined amino acid residues) in the encoded neublastin polypeptide. Those skilled in the art recognize that site-directed mutagenesis is a routine and widely-used technique. In fact, many site-directed mutagenesis kits are commercially available. One such kit is the "Transformer Site Directed Mutagenesis Kit" sold by Clontech Laboratories (Palo Alto, Calif.).

Practice of the present invention will employ, unless indicated otherwise, conventional techniques of cell biology, cell culture, molecular biology, microbiology, recombinant DNA, protein chemistry, and immunology, which are within the skill of the art. Such techniques are described in the literature. See, for example, Molecular Cloning: A Laboratory Manual, 2nd edition. (Sambrook, Fritsch and Maniatis, eds.), Cold Spring Harbor Laboratory Press, 1989; DNA Cloning, Volumes I and II (D.N. Glover, ed), 1985; Oligonucleotide Synthesis, (M.J. Gait, ed.),1984; U.S. Patent No. 4,683,195 (Mullis et al.,); Nucleic Acid Hybridization (B.D. Haines and S.J, Higgins, eds.), 1984; Transcription and Translation (B.D. Hames and S.J. Higgins, eds.), 1984; Culture of Animal Cells (R.I. Freshney, ed). Alan R. Liss, Inc., 1987; Immobilized Cells and Enzymes, IRL Press, 1986; A Practical Guide to Molecular Cloning (13.Perbal), 1984; Methods in Enzymology, Volumes 154 and 155 (Wu et al., eds), Academic Press, New York; Gene Transfer Vectors for Mammalian Cells (J.H. Miller and M.P. Calos, eds.), 1987, Cold Spring Harbor Laboratory; Immunochemical Methods in Cell and Molecular Biology (Mayer and Walker, eds.), Academic Press, London, 1987; Handbook of Experiment Immunology, Volumes I-IV (D.M. Weir and C.C. Blackwell, eds.), 1986; Manipulating the Mouse Embryo, Cold Spring Harbor Laboratory Press, 1986.

### Variant neublastin fusion proteins

If desired, the variant neublastin polypeptide can be provided as a fusion protein. Fusion polypeptide derivatives of proteins of the invention also include various structural forms of the primary protein which retain biological activity. As used herein "fusion" refers to a co-linear, covalent linkage of two or more proteins or fragments thereof via their individual peptide backbones, most preferably through genetic expression of a polynucleotide molecule encoding those proteins in the same reading frame (i.e., "in frame"). It is preferred that the proteins or fragments thereof are from different sources. Thus, preferred fusion proteins include a variant neublastin protein or fragment covalently linked to a second moiety that is not a variant neublastin. Preferably, the second moiety is derived from a polypeptide that exists as a monomer, and is sufficient to confer enhanced solubility and/or bioavailability properties on the neublastin polypeptide.

For example, a "variant neublastin/human serum albumin fusion" is a protein comprising a variant neublastin polypeptide of the invention, or fragment thereof, whose N-terminus or C-tenniims is linked in frame to a human serum albumin polypeptide (See Syed et al., Blood, 1997,89: 3243 and Yeh et al., P.N.A.S. USA 1992, 89:1904) and US Patent Nos. 5,876,969 and 5,302,697. The term "fusion protein" additionally includes a variant neublastin chemically linked via a mono- or hetero- functional molecule to a second moiety that is not a variant neublastin protein and is made de novo from purified protein as described below.

Neublastin-serum albumin fusions can be constructed using methods known in the art. Any of a number of cross-linkers that contain a corresponding amino reactive group and thiol reactive group can be used to link neublastin to serum albumin. Examples of suitable linkers include amine reactive cross-linkers that insert a thiol reactive-maleimide. These include, *e.g.,* SMCC, AMAS, BMPS, MBS, EMCS, SMPB, SMPH, KMUS, or GMBS. Other suitable linkers insert a thiol reactive-haloacetate group. These include, e.g., SBAP, SIA, SIAB and that provide a protected or non protected thiol for reaction with sulfhydryl groups to product a reducible linkage are SPDP, SMPT, SATA, or SATP all of which are commercially available (e.g., Pierce Chemicals). One skilled in the art can similarly envision with alternative strategies that will link the N-terminus of neublastin with serum albumin.

It is also envisioned that one skilled in the art can generate conjugates to serum albumin that are not targeted at the N-terminus of NBN or at the thiol moiety on serum albumin. If desired, NBN-serum albumin fusions can be generated using genetic engineering techniques, wherein NBN is fused to the serum albumin gene at its N-terminus, C-terminus, or at both ends.

It is further contemplated that any NBN conjugate that results in a product with a prolonged half life in animals (including humans) can be generated using a similar strategy.

Other derivatives of variant neublastins include covalent or aggregate conjugates of variant neublastin or its fragments with other proteins or polypeptides, such as by synthesis in recombinant culture as additional N-termini, or C-termini. For example, the conjugated peptide may be a signal (or leader) polypeptide sequence at the N-terminal region of the protein which co-translationally or post-translationally directs transfer of the protein from its site of synthesis to its site of function inside or outside of the cell membrane or wall (e.g., the yeast alpha -factor leader). Neublastin receptor proteins can comprise peptides added to facilitate purification or identification of neublastin (e.g., histidine/neublastin fusions). The amino acid sequence of neublastin can also be linked to the peptide Asp-Tyr-Lys-Asp-Asp-Asp-Asp-Lys (DYKDDDDK) (Hopp et al., Biotechnology 6:1204,1988.) The latter sequence is highly antigenic and provides an epitope reversibly bound by a specific monoclonal antibody, enabling rapid assay and facile purification of expressed recombinant protein.

This sequence is also specifically cleaved by bovine mucosal enterokinase at the residue immediately following the Asp-Lys pairing.

### Variant neublastin polypeptides conjugated to a polymer

If desired, a single polymer molecule may be employed for conjugation with a neublastin polypeptide, although it is also contemplated that more than one polymer molecule can be attached as well. Conjugated neublastin compositions of the invention may find utility in both in vivo as well as non-in vivo applications. Additionally, it will be recognized that the conjugating polymer may utilize any other groups, moieties, or other conjugated species, as appropriate to the end use application. By way of example, it may be useful in some applications to covalently bond to the polymer a functional moiety imparting UV-degradation resistance, or antioxidation, or other properties or characteristics to the polymer. As a further example, it may be advantageous in some applications to functionalize the polymer to render it reactive or cross-linkable in character, to enhance various properties or characteristics of the overall conjugated material. Accordingly, the polymer may contain any functionality, repeating groups, linkages, or other constituent structures which do not preclude the efficacy of the conjugated neublastin mutein composition for its intended purpose.

Illustrative polymers that may usefully be employed to achieve these desirable characteristics are described herein below in exemplary reaction schemes. In covalently bonded peptide applications, the polymer may be functionalized and then coupled to free amino acid(s) of the peptide(s) to form labile bonds.

In some embodiments, the neublastin polypeptide is linked to the polymer via a terminal reactive group on the polypeptide. Alternatively, or in addition, the neublastin polypeptide may be linked via the side chain amino group of an internal lysine residue, *e.g.,* a lysine residue introduced into the amino acid sequence of a naturally occurring neublastin polypeptide. Thus, conjugations can also be branched from the non terminal reactive groups. The polymer with the reactive group(s) is designated herein as "activated polymer". The reactive group selectively reacts with free amino or other reactive groups on the protein.

Attachment may occur in the activated polymer at any available neublastin amino group such as the alpha amino groups or the epsilon amino groups of a lysine residue or residues introduced into the amino acid sequence of a neublastin polypeptide or variant thereof. Free carboxylic groups, suitably activated carbonyl groups, hydroxyl, guanidyl, imidazole, oxidized carbohydrate moieties and mercapto groups of the neublastin (if available) can also be used as attachment sites.

Generally from about 1.0 to about 10 moles of activated polymer per mole of protein, depending on protein concentration, is employed. The final amount is a balance between maximizing the extent of the reaction while minimizing non-specific modifications of the product and, at the same time, defining chemistries that will maintain optimum activity, while at the same time optimizing, if possible, the half-life of the protein. Preferably, at least about 50% of the biological activity of the protein is retained, and most preferably near 100% is retained.

The reactions may take place by any suitable method used for reacting biologically active materials with inert polymers, preferably at about pH 5-8, e.g., pH 5, 6, 7, or 8, if the reactive groups are on the alpha amino group at the N-terminus. Generally the process involves preparing an activated polymer and thereafter reacting the protein with the activated polymer to produce the soluble protein suitable for formulation. The above modification reaction can be performed by several methods, which may involve one or more steps.

The polymer can be coupled to the variant neublastin polypeptide using methods known in the art. For example, in one embodiment, the polyalkylene glycol moiety is coupled to a lysine group of the neublastin polypeptide or variant neublastin polypeptide. Linkage to the lysine group can be performed with a N-hydroxylsuccinimide (NHS) active ester such as PEG succinimidyl succinate (SS-PEG) and succinimidyl propionate (SPA-PEG). Suitable polyalkylene glycol moieties include, *e.g.,* carboxymethyl-NHS, norleucine-NHS, SC-PEG, tresylate, aldehyde, epoxide, carbonylimidazole, and PNP carbonate.

Additional amine reactive PEG linkers can be substituted for the succinimidyl moiety. These include, e.g. isothiocyanates, nitrophenylcarbonates, epoxides, and benzotriazole carbonates. Conditions are preferably chosen to maximize the selectivity and extent or reaction. Linear and branched forms of PEG can be used as well as other alkyl forms. The length of the PEG can be varied. Most common forms vary in size from 2K-100K. While the present examples report that targeted pegylation at the N-terminus does not affect pharmokinetic properties, the fact that the material retained physiological function indicates that modification at the site or sites disclosed herein is not deleterious. Consequently, in generating mutant forms of NBN that could provide additional sites of attachment through insertion of lysine residues, the likely outcome that these forms would be pegylated both at the lysine and at the N-terminus is considered acceptable.

If desired, neublastin variant polypeptides may contain a tag, e.g., a tag that can subsequently be released by proteolysis. Thus, the lysine moiety can be selectively modified by first reacting a his-tag variant with a low molecular weight linker such as Traut's reagent (Pierce) which will react with both the lysine and N-terminus, and then releasing the his tag. The polypeptide will then contain a free SH group that can be selectively modified with a PEG containing a thiol reactive head group such as a maleimide group, a vinylsulfone group, a haloacetate group, or a free or protected SH.

Traut's reagent can be replaced with any linker that will set up a specific site for PEG attachment, By way of example, Traut's reagent could be replaced with SPDP, SMPT, SATA, or SATP (all available from Pierce). Similarly one could react the protein with a amine reactive linker that inserts a maleimide (for example SMCC, AMAS, BMPS, MBS, EMCS, SMPB, SMPH, KMUS, or GMBS), a haloacetate group (SBAP, SIA, SIAB), or a vinylsulfone group and react the resulting product with a PEG that contains a free SH. The only limitation to the size of the linker that is employed is that it cannot block the subsequent removal of the N-terminal tag.

Thus, in other embodiments, the polyalkylene glycol moiety is coupled to a cysteine group of the neublastin polypeptide or variant neublastin polypeptide. Coupling can be effected using, e.g., a maleimide group, a vinylsulfone group, a haloacetate group, and a thiol group.

One or more sites on a variant neublastin polypeptide can be coupled to a polymer. For example, one two, three, four, or five PEG moieties can be attached to the polymer. In some embodiments, a PEG moiety is attached at the amino terminus and/or amino acids 14, 39, 68, and 95 of a neublastin polypeptide numbered as shown in Table 1.

In preferred embodiments, the variant neublastin polypeptide in the composition has a longer serum half-life relative to the half-life of the variant polypeptide in the absence of the polymer. Alternatively, or in addition, the variant neublastin polypeptide in the composition binds GFRα3, activates RET, normalizes pathological changes of a neuron, or enhances survival of a neuron, or performs a combination of these physiological functions.

In preferred embodiments, the composition is provided as a stable, aqueously soluble conjugated neublastin polypeptide or variant neublastin polypeptide complex comprising a neublastin polypeptide or variant neublastin polypeptide coupled to a polyethylene glycol moiety. If desired, the neublastin polypeptide or variant neublastin polypeptide may be coupled to the polyethylene glycol moiety by a labile bond. The labile bond can be cleaved in, *e.g.*, biochemical hydrolysis, proteolysis, or sulfhydryl cleavage. For example, the bond can be cleaved under in vivo (physiological) conditions.

### Pharmaceutical compositions containing variant neublastin-polymer conjugates

Also provided is a pharmaceutical composition including a variant neublastin-polymer conjugate of the present invention. A "pharmaceutical composition" as used herein is defined as comprising a neublastin protein or conjugate of the invention, dispersed in a physiologically acceptable vehicle, optionally containing one or more other physiologically compatible ingredients. The pharmaceutical composition thus may contain an excipient such as water, one or more minerals, sugars, detergents, and one or more carriers such as an inert protein (e.g., heparin or albumin).

The polymer-neublastin conjugates of the invention may be administered per se as well as in the form of pharmaceutically acceptable esters, salts, and other physiologically functional derivatives thereof. In such pharmaceutical and medicament formulations, the variant neublastin conjugate preferably is utilized together with one or more pharmaceutically acceptable earrier(s) and optionally any other therapeutic ingredients.

The carrier(s) must be pharmaceutically acceptable in the sense of being compatible with the other ingredients of the formulation and not unduly deleterious to the recipient thereof. The variant neublastin is provided in an amount effective to achieve a desired pharmacological effect or medically beneficial effect, as described herein, and in a quantity appropriate to achieve the desired bioavailable in vivo dose or concentration.

The formulations include those suitable for parenteral as well as non parenteral administration, and specific administration modalities include oral, rectal, buccal, topical, nasal, ophthalmic, subcutaneous, intramuscular, intravenous, transdermal, intrathecal, intra-articular, intra-arterial, sub-arachnoid, bronchial, lymphatic, vaginal, and intra-uterine administration. Formulations suitable for aerosol and parenteral administration, both locally and systemically, are preferred.

When the variant neublastin is utilized in a formulation comprising a liquid solution, the formulation advantageously may be administered orally, bronchially, or parenterally. When the neublastin is employed in a liquid suspension formulation or as a powder in a biocompatible carrier formulation, the formulation may be advantageously administered orally, rectally, or bronchially. Alternatively, it may be administered nasally or bronchially, via nebulization of the powder in a carrier gas, to form a gaseous dispersion of the powder which is inspired by the patient from a breathing circuit comprising a suitable nebulizer device.

The formulations comprising the proteins of the present invention may conveniently be presented in unit dosage forms and may be prepared by any of the methods well known in the art of pharmacy. Such methods generally include the step of bringing the active ingredient(s) into association with a carrier which constitutes one or more accessory ingredients.

Typically, the formulations are prepared by uniformly and intimately bringing the active ingredient(s) into association with a liquid carrier, a finely divided solid carrier, or both, and then, if necessary, shaping the product into dosage forms of the desired formulation.

Formulations of the present invention suitable for oral administration may be presented as discrete units such as capsules, cachets, tablets, or lozenges, each containing a predetermined amount of the active ingredient as a powder or granules; or a suspension in an aqueous liquor or a non-aqueous liquid, such as a syrup, an elixir, an emulsion, or a draught.

Formulations suitable for parenteral administration conveniently comprise a sterile aqueous preparation of the active conjugate, which preferably is isotonic with the blood of the recipient (e.g., physiological saline solution). Such formulations may include suspending agents and thickening agents or other microparticulate systems which are designed to target the compound to blood components or one or more organs. The formulations may be presented in unit-dose or multi-dose form.

Nasal spray formulations comprise purified aqueous solutions of the active conjugate with preservative agents and isotonic agents. Such formulations are preferably adjusted to a pH and isotonic state compatible with the nasal mucus membranes.

Formulations for rectal administration may be presented as a suppository with a suitable carrier such as cocoa butter, hydrogenated fats, or hydrogenated fatty carboxylic acid. Ophthalmic formulations such as eye drops are prepared by a similar method to the nasal spray, except that the pH and isotonic factors are preferably adjusted to match that of the eye.

Topical formulations comprise the conjugates of the invention dissolved or suspended in one or more media, such as mineral oil, petroleum, polyhydroxy alcohols, or other bases used for topical pharmaceutical formulations.

In addition to the aforementioned ingredients, the formulations of this invention may further include one or more accessory ingredient(s) selected from diluents, buffers, flavoring agents, disintegrants, surface active agents, thickeners, lubricants, preservatives (including antioxidants), and the like. The foregoing considerations apply also to the neublastin fusion proteins of the invention (e.g., neublastin-HSA fusions).

Accordingly, the present invention contemplates the provision of suitable fusion proteins for in vitro stabilization of a variant neublastin conjugate in solution, as a preferred illustrative application of the invention. The fusion proteins may be employed for example to increase the resistance to enzymatic degradation of the variant neublastin polypeptide and provides a means of improving shelf life, room temperature stability, and the like. It is understood that the foregoing considerations apply also to the neublastin-serum albumin fusion proteins (including the human neublastin-human serum albumin fusion proteins) of the invention.

### Methods of treatment

The variant neublastin polypeptides, as well as fusion proteins, or conjugates thereof, may be used for treating or alleviating a disorder or disease of a living animal body, preferably of a mammal, more preferably a primate including a human, which disorder or disease is responsive to the activity of neurotrophic agents.

The compositions of the invention may be used directly via, e.g., injected, implanted or ingested pharmaceutical compositions to treat a pathological process responsive to the neublastin polypeptides. The compositions may be used for alleviating a disorder or disease of a living animal body, including a human, which disorder or disease is responsive to the activity of neurotrophic agents. The disorder or disease may in particular be damage of the nervous system caused by trauma, surgery, ischemia, infection, metabolic diseases, nutritional deficiency, malignancy or toxic agents, and genetic or idiopathic processes.

The damage may in particular have occurred to sensory neurons or retinal ganglion cells, including neurons in the dorsal root ganglia of the spinal cord or in any of the following tissues: the geniculate, petrosal and nodose ganglia; the vestibuloacoustic complex of the eighth cranial nerve; the ventrolateral pole of the maxillomandribular lobe of the trigeminal ganglion; and the mesencephalic trigeminal nucleus.

In a preferred embodiment of the method of the invention, the disease or disorder is a neurodegenerative disease involving lesioned and traumatic neurons, such as traumatic lesions of peripheral nerves, the medulla, and/or the spinal cord, cerebral ischemic neuronal damage, neuropathy and especially peripheral neuropathy, peripheral nerve trauma or injury, ischemic stroke, acute brain injury, acute spinal cord injury, nervous system tumors, multiple sclerosis, exposure to neurotoxins, metabolic diseases such as diabetes or renal dysfunctions and damage caused by infectious agents, neurodegenerative disorders including Alzheimer's disease, Huntington's disease, Parkinson's disease, Parkinson-Plus syndromes, progressive Supranuclear Palsy (Steele-Richardson-Olszewski Syndrome), Olivopontocerebellar Atrophy (OPCA), Shy-Drager Syndrome (multiple systems atrophy), Guamanian parkinsonism dementia complex, amyotrophic lateral sclerosis, or any other congenital or neurodegenerative disease, and memory impairment connected to dementia.

In a preferred embodiment, sensory and/or autonomic system neurons can be treated. In particular, nociceptive and mechanoreceptive neurons can be treated, more particularly delta fiber and C-fiber neurons. In addition, sympathetic and parasympathetic neurons of the autonomic system can be treated.

In another preferred embodiment, motor neuron diseases such as amyotrophic lateral sclerosis ("ALS") and spinal muscular atrophy can be treated. In yet another preferred embodiment, the neublastin molecules of this invention to can be used to enhance nerve recovery following traumatic injury. Alternatively, or in addition, a nerve guidance channel with a matrix containing variant neublastin polypeptides, or fusion or conjugates of variant neublastin polypeptides can be used in the herein described methods. Such nerve guidance channels are disclosed, e.g., United States Patent No. 5,834,029, incorporated herein by reference.

In a preferred embodiment, the compositions disclosed herein (and pharmaceutical compositions containing same) are used in the treatment of peripheral neuropathies. Among the peripheral neuropathies contemplated for treatment with the molecules of this invention are trauma-induced neuropathies, e.g., those caused by physical injury or disease state, physical damage to the brain, physical damage to the spinal cord, stroke associated with brain damage, and neurological disorders related to neurodegeneration. Also included herein are those neuropathies secondary to infection, toxin exposure, and drug exposure. Still further included herein are those neuropathies secondary to systemic or metabolic disease. The herein disclosed compositions can also be used to treat chemotherapy-induced neuropathies (such as those caused by delivery of chemotherapeutic agents, e.g., taxol or cisplatin); toxin induced neuropathies, drug-induced neuropathies, vitamin-deficiency induced neuropathies; idiopathic neuropathies; and diabetic neuropathies. See, e. g., United States patents 5,496,804 and 5,916,555, each herein incorporated by reference.

Additional conditions that can be treated using the herein described compositions are mono-neuropathies, mono-multiplex neuropathies, and poly-neuropathies, including axonal and demyelinating neuropathies, using the herein described compositions.

In another preferred embodiment, the compositions of the invention (and pharmaceutical compositions containing same) are used in the treatment of various disorders in the eye, including photoreceptor loss in the retina in patients afflicted with macular degeneration, retinitis pigmentosa, glaucoma, and similar diseases.

The invention will be further illustrated in the following non-limiting examples.

### Example 1--Bioavailability of N-terminal pegylated neublastin

CHO cell derived recombinant human neublastin was observed to be rapidly cleared from circulation if administered intravenously in rats. None of the protein was detected in the serum following subcutaneous administration. To increase bioavailability of neublastin, pegylated forms were constructed.

Because no lysines occur in the NBN sequence, amine-specific pegylation chemistries will result in pegylation of an NBN polypeptide at its amino terminus. Thus, for each neublastin dimer, two PEG moieties should be attached. Accordingly, PEG forms were first directly targeted to the N-terminus through amine specific chemistries. Surprisingly, pegylation even with two, 20 K PEGs attached had little benefit on half life, indicating that a mechanism based clearance pathway was overriding the enhancement in half life that was expected to be achieved by pegylation.

### Example 2--Construction of a pegylated neublastin N95K mutein

The bioavailability of NBN mutant forms pegylated at internal amino acid residues was next examined. A series of four mutants replacing naturally occurring residues at positions 14,39,68, and 95 were designed to insert lysines at selected sites in the sequence. These lysines would provide alternative sites for PEG attachment. These sites were selected using the crystal structure of GDNF (Nat.Struct.Biol. 4: 435-8, 1997) as a framework to identify surface residues and the persephin/ neublastin chimera mutagenesis study (J.Biol.Chem. 275: 3412-20, 2000) to identify functionally important regions of the structure that should be avoided.

Two of the mutations (R39 and R68) were targeted at a region that based on the distribution of positive charges on the surface might represent a heparin binding site, a property of the protein which likely contributes to its rapid clearance. A third site was targeted at N95, the natural glycosylation site in wild-type NBN. This site is naturally modified with a complex carbohydrate structure. Therefore, modification with PEG at this site was not expected to impact function. The fourth site (R14) was selected in a region that was not covered by any other of the modifications. A mutant in which the asparagine residue at position 95 was replaced with a lysine (the "N95K mutant") was chosen for the studies disclosed herein.

Four different rat neublastin muteins containing one or more alterations in the wild-type sequence of rat neublastin polypeptide were constructed. The muteins include the single N95K mutein, and the muteins containing single substitutions at other sites in the amino acid sequence of rat neublastin: R14K; R68K; and R39K. In the "X₁N₁X₂" nomenclature, X₁ refers to an amino acid of a wild-type neublastin polypeptide, N₁, refers to the numerical position of the X₁ amino acids in the sequence, as numbered according to SEQ ID NO: 1. X₂ refers to an amino acid substituted for the wild-type amino acid at the indicated numerical position in the sequence.
To construct the rat N95K neublastin mutation, site-directed mutagenesis was performed on pCMB020, a plasmid encoding wild-type rat neublastin. The wild-type rat neublastin nucleic and the amino acid sequence of the polypeptide encoded thereby are presented below:

1 ATGGAACTGG GACTTGGAGA GCCTACTGCA TTGTCCCACT GCCTCCGGCC

51 TAGGTGGCAA CCAGCCTTGT GGCCAACCCT AGCTGCTCTA GCCCTGCTGA

101 GCAGCGTCAC AGAAGCTTCC CTGGACCCAA TGTCCCGCAG CCCCGCCTCT

151 CGCGATGTTC CCTCGCCGGT CCTGGCGCCC CCAACAGACT ACCTACCTGG

201 GGGACACACC GCACATCTGT GCAGCGAAAG AGCCCTGCGA CCACCGCCGC

251 AGTCTCCTCA GCCCGCACCC CCACCACCGG GTCCCGCGCT CCAGTCTCCT

301 CCCGCTGCGC TCCGCGGGGC ACGCGCGGCG CGTGCAGGAA CCCGGAGCAG

351 CCGCGCACGG GCTACAGATG CGCGCGGCTG CCGCCTGCGC TCACAGCTGG

401 TGCCGGTGAG CGCTCTCGGC CTGGGCCACA GCTCCGACGA GCTGATACGT

451 TTCCGCTTCT GCAGCGGTTC GTGCCGCCGA GCACGCTCCC CGCACGATCT

501 CAGCCTGGCC AGCCTGCTGG GCGCCGGGGC CCTGCGGTCT CCTCCCGGGT

551 CCCGGCCGAT CAGCCAGCCC TGTTGCCGGC CCACTCGCTA TGAGGCAGTC

601 TCCTTCATGG ACGTGAACAG CACCTGGAGA ACCGTGGACC ATCTCTCCGC

651 CACCGCCTGC GGCTGTCTGG GCTGA (SEQ ID NO:5)

1     MELGLGEPTA LSHCLRPRWQ PALWPTLAAL ALLSSVTEAS LDPMSRSPAS

51     RDVPSPVLAP PTDYLPGGHT AHLCSERALR PPPQSPQPAP PPPGPALQSP

101  PAALRGARAA RAGTRSSRAR ATDARGCRLR SQLVPVSALG LGHSSDELIR

151  FRFCSGSCRR ARSPHDLSLA SLLGAGALRS PPGSRPISQP CCRPTRYEAV

201  SFMDVNSTWR TVDHLSATAC GCLG* (SEQ ID NO:4)

Mutagenesis of pCM020 using oligonucleotides KD2-310 and KD3-211 resulted in formation of the plasmid pCMB027:
KD2-310 5'-GTATCTTTCATGGACGTAAAGTCTACATGGAGAACCGTAGATCATCTATCTGCAACC-3' (SEQ ID NO:6)
KD2-311 5'-GGTTGCAGATAGATGATCTACGGTTCTCCATGTAGACTTTACGTCCATGAAAGATAC-3' (SEQ ID NO:7)

In pCMB027, the codon encoding asparagine at position 95 has been replaced with a codon encoding lysine.

A R14K neublastin mutein formed by replacement of a codon encoding arginine at position 14 with a codon encoding lysine in the neublastin coding sequence of pCMB020. Site-directed mutagenesis was performed on pCMB020 using oligonucleotides KD3-254 and KD3-255:
KD3-254 5'-GCTGGAACTCGCAGCTCTCGTGCTCGTGCAACGGATGCAAAAGGGTGTCG-3' (SEQ ID NO:8)
KD2-255 5'-CGACAGCCTTTTGCATCCGTTGCACGAGCACGAGAGCTGCGAGTTCCAGC-3' (SEQ ID NO:9)

The resulting construct was named pCMB029.

A R68K neublastin mutein formed by replacement of a codon encoding arginine at position 68 with a codon encoding lysine in the neublastin coding sequence of pCMB020. Site-directed mutagenesis was performed on pCMB020 using oligonucleotides KD3-258 and KD3-259:
KD3-258 5'-GGAGCCGGAGCACTAAARTCTCCCCGGGATCTAGACC-3' (SEQ ID NO:10)
KD3-259 5'-GGTCTAGATCCCGGGGGAGATTTTAGTGCTCCGGCTCC-3' (SEQ ID NO:11)

The resulting construct was named pCMB030.

A R39K neublastin mutein formed by replacement of arginine at amino acid 39 with lysine in the neublastin coding sequence of pCMB020. Site-directed mutagenesis of pCMB027 was performed using oligonucleotides KD3-256 and KD3-257:
KD3-256 5'-GACGAATTAATTAAGTTTCGTTTTTGTTCAGG-3' (SEQ ID NO:12)
KD3-257 5'-CCTGAACAAAAACGAAACTTAATTAATTCGTC-3' (SEQ ID NO:13)

For expression and purification, a plasmid encoding the rat NBN N95K mutein was expressed in *E.coli* as a His-tagged fusion protein with an enterokinase cleavage site immediately adjacent to the start of the mature 113 amino acid NBN sequence. The *E. coli* was grown in a 500 L fermentor and frozen cell paste was provided. The *E.coli* cells were lysed in a Manton Gaulin Press and the rat NBN NK recovered from the insoluble washed pellet fraction.

The NBN was extracted from the pellet with guanidine hydrochloride, refolded, and the his-tag removed with enterokinase. The product was then subjected to chromatography on Ni NTA agarose (Qiagen) and on Bakerbond WP CBX cation exchange resin.

Enterokinase treatment of the his tagged product resulted in an aberrant cleavage of the protein at arginine 7 in the mature sequence. The resulting des 1-7 NBN product was fully active in the KIRA ELISA and structurally indistinguishable from the mature form in its susceptibility to guanidine-induced denaturation and therefore was used for subsequent work.

Rat NBN N95K was pegylated at an average of 3.3 PEG moieties/molecule using methoxylpoly(ethylene glycol)-succinimidyl propionate (SPA-PEG) with a molecular mass of 10,000 Da as the reactant. The resulting pegylated product was subjected to extensive characterization including analysis by SDS-PAGE, size exclusion chromatography (SEC), reverse phase HPLC, matrix assisted laser desorption/ionization mass spectrometry (MALD/IMS), peptide mapping, assessment of activity in the KIRA ELISA, and determination of endotoxin content. The purity of the NBN N95K product prior to pegylation as measured by SDS-PAGE and SEC was greater than 95%. The NBN N95K product migrated under nonreducing conditions as a dimer, consistent with its predicted structure. After pegylation, the resulting product consisted of a series of modified adducts containing 2 PEGs/ molecule 5% of the product, 3 PEGs/molecule 60% of the product, 4 PEGs/molecule 30% of the product, and several minor forms of higher mass. In the pegylated sample there was no evidence of aggregates. Residual levels of unmodified NBN in the product were below the limits of quantitation. The endotoxin content of the material is routinely less than 1 EU/mg. The specific activity of the pegylated NBN in the KIRA ELISA is 10 nM. The pegylated product was formulated at 1.1 mg/mL in PBS pH 6.5. The material, which is similar in potency to wt-NBN, can be supplied as a frozen liquid, which is stored at -70°C.

The R14K, R39K, and R68K muteins were expressed in E. coli and can be subjected to the same methods for purification, pegylation and assessment of function as described above for the N95K-NBN.

### Preparation of pegylated NBN

230 mL of the refolded rat NBN N95K (2.6 mg/mL) that had been produced in E.coli and stored at 4°C in 5 mM sodium phosphate pH 6.5, 100 mM NaCl was diluted with 77 mL of water, 14.4 mL of 1M HEPES pH7.5, and 2.8 g (10 mg/mL final) of PEG SPA 10,000 Da (Shearwater Polymers, Inc.). The sample was incubated at room temperature for 4 hours in the dark, then treated with 5 mM imidazole (final), filtered, and stored overnight at 4°C. The product was generated in two batches one containing 130 mL of the NBN N95K bulk and the other containing 100 mL of the bulk. The pegylated NBN was purified from the reaction mixture on Fractogel EMD SO₃⁻ (M) (EM Industries). The column was run at room temperature. All buffers were prepared pyrogen free. Sodium chloride was added to the reaction mixture to a final concentration of 87 mM and the sample was loaded onto a 45 mL Fractogel column (5 cm internal diameter).

The column was washed with one column volume of 5 mM sodium phosphate pH 6.5, 80 mM NaCl, then with three one column volume aliquots of 5 mM sodium phosphate containing 50 mM NaCl. The resin was transferred into a 2.5 cm diameter column and the pegylated NBN was eluted from the column with six ten mL steps containing 5 mM sodium phosphate pH 6.5, 400 mM NaCl, three steps containing 500 mL NaCl, and six steps containing 600 mM NaCl. Elution fractions were analyzed for protein content by absorbance at 280 nm and then for extent of modification by SDS-PAGE. Selected fractions were pooled, filtered through a 0. 2 µm filter, and diluted with water to 1.1 mg pegylated rat NBN/mL. After assessing endotoxin levels in the individual batches, they were pooled and refiltered through a 0.2 µm membrane. The final material was aliquoted and stored at - 70°C.

### UV spectrum of purified pegylated NBN NK

The UV spectrum (240-340 nm) of pegylated NBN NK was taken on the neat sample. The sample was analyzed in triplicate. The pegylated sample exhibited an absorbance maximum at 275-277 nm and an absorbance minimum at 247-249. This result is consistent with what is observed on the unmodified NBN bulk intermediate. The protein concentration of the pegylated product was estimated from the spectrum using an extinction coefficient of Σ₂₈₀^{0.1%}=0.50. The protein concentration of the pegylated NBN bulk is 1.1 mg/mL. No turbidity was present in the sample as evident by the lack of absorbance at 320 nm.

### Characterization of pegylated NBN NK by SDS-PAGE

Aliquots of pegylated NBN containing 3, 1.5, 0.75, and 0.3 µg of the product were subjected to SDS-PAGE on a 4-20% gradient gel (Owl). The gel was stained with Coomassie brilliant blue R-250. Molecular weight markers (GTBCO-BRL) were run in parallel.

SDS-PAGE analysis of pegylated NBN NK under non reducing conditions revealed a series of bands corresponding to modifications with 2, 3, 4, and more than 4 PEGs/ molecule. The major band with apparent mass of 98 kDa contains 3 PEGS/ molecules. In the purified, pegylated product unmodified NBN was not detected. The presence of a mixture of products with 2, 3 and 4 PEGS attached was verified by MALDI mass spectrometric analysis. The ratio of product containing 2, 3, and 4 PEGs was determined by densitometry and determined to be 7, 62, and 30 percent of the total, respectively.

### Characterization of pegylated NBN by size exclusion chromatography

Pegylated NBN was subjected to size exclusion chromatography on an analytical Superose 6 HR1O/30 FPLC column using 5 mM MES pH 6.5, 300 mM NaCl as the mobile phase. The column was run at 20 mL/h. Elution fractions were monitored for absorbance at 280 nm. The pegylated NBN eluted as a single peak with an apparent molecular weight of about 200 kDa consistent with the large hydrodynamic volume of the PEG. No evidence of aggregates were observed. Free NBN, which elutes with an apparent molecular mass of about 30kDa, was not detected in the preparation.

### Analysis of pegylated NBN by reverse phase HPLC

Pegylated NBN NK was subjected to reverse phase HPLC on a Vydac C₄ (5 µm, 1 x 250 mm) column. The column was developed using a 60 mm gradient from 40 to 60% B (Buffer A: 0.1% TFA, Buffer B: 75% acetonitrile/0.085% TFA). The column effluent was monitored for absorbance at 214 nm and fractions collected for subsequent analysis. Pegylated NBN NK was fractionated into its various di (60.5 mm), tri (63.3 mm), and tetra (67.8 mm) pegylated components by reverse phase HPLC on a C₄ column. The relative intensities of the peaks suggest that the ratios of the components are 5.4, 60.5, and 30.1 %, respectively. Peak identities were verified by MALDI-MS. There was no evidence of non pegylated NBN NK (elutes at 5- 15 mm) in the product.

### Analysis of pegylated NBN by mass spectrometry

Pegylated NBN NK was desalted on a C₄ Zip Tip and analyzed by mass spectrometry on a Voyager-DE™ STR (PerSeptive Biosystems) matrix-assisted laser desorption/ionization time-of- flight (MALDI-TOF) mass spectrometer using sinapinic acid as a matrix. 0.5 uL of the purified protein was mixed with 0.5 uL of matrix on the target plate. Mass spectrometry of pegylated NBN revealed singly and doubly charged forms of three adducts. The observed masses of 43803 Da, 54046 Da, and 64438 Da are consistent with modifications of 2, 3, and 4 PEGs/ molecule.

### Analysis of pegylated NBN by peptide mapping.

The specificity of the pegylation reaction was evaluated by peptide mapping. Pegylated NBN was separated into di, tri, and tetra pegylated components, which were then reduced, alkylated, and further separated into their single chain components by HPLC on a C₄ column. These components and reduced and alkylated unmodified NBN NK as a control were digested with Asp-N proteinase and the resulting cleavage products were fractionated by reversed phase HPLC on a Vydac C₁₈ (5 µm, 1 x 250 mm) column using a 60 mm gradient from 0 to 60% B (Buffer A: 0.1% TFA, Buffer B: 75% acetonitrile/0.085% TFA). The column effluent was monitored for absorbance at 214 mm.

The rat NBN sequence contains four internal aspartic acids and therefore was expected to yield a simple cleavage profile when digested with endoproteinase Asp-N. All of the peaks from the Asp-N digest of rat NBN NK have been identified by mass spectrometry and/or Edman N-terminal sequencing and thus the peptide map can be used as a simple tool to probe for the sites of modification by the presence or absence of a peak. The identity of the various peaks are summarized below in Table 3

**Table 3**

| Peak by Retention Time (mm) | Observed Mass Average | Theoretical Mass Average | Assignment | Amino Acid Sequence |
|---|---|---|---|---|
| 38.8 | 1261.1 (M) | 1262.4 | 102-113 | DHLSATACGCLG |
| 40.7 | 1090.9 | 1092.2 | 93-101 | DVKSTWRTV |
| 44.6 | 2508.4 | 2508.9 | 35-54 | DELIRFRFCSGSCRRARSPH |
| 46.0 | 2437.0 | 2437.8 | 12-34 | DARGCRLRSQLVPVSALGLGHSS |
| 51.4 | 3456.7 | 3456.0 | 55-86 | DLSLAS.....CRPTRY |
| 51.9 | 4134.4 | | 55-92(oxid) | DLSLAS CRPTRYEAVSFM |
| 53.2 | 4136.3 * | 4120.8 | 55-92 | DLSLAS CRPTRYEAVSFM |

| | | | | |
|---|---|---|---|---|
| (M): monolostopic mass *: due to oxidation of methionine containing peptide on MALDI | | | | |

Since NBN exists as a homodimer, the rat NBN NK product contains four potential sites for pegylation, the two N-terminal amines from each of the chains and the two N95K sites that were engineered into the construct. In the peptide map of the dipegylated chain, only the peak that contains the peptide with the NK mutation was altered by the PEG modification. None of the other peaks were affected by the PEG modification. The mapping data therefore indicate that the PEG moiety is specifically attached to this peptide and not to any of the other peptides that were screened. The second potential site of attachment, the N-terminus is on a peptide that is only three amino acids long and is not detected in the peptide map. It is inferred that additional PEG attachments are at this site. Consistent with this notion, a small percentage of the rat NBN NK is not truncated and contains the mature Ala-1 sequence. This peptide elutes at 30 µm and is visible in the peptide map from the non-pegylated digest, but is absent from the pegylated NBN NK digests.

### Example 3. Assessing the potency of internally pegylated neublastin in a kinase receptor activation (KIRA) ELISA

The potency of pegylated rat NBN was measured using NBN dependent activation/phosphorylation of c-Ret as a reporter for NBN activity in an ELISA that was specific for the presence of phosphorylated RET. NB41A3-mRL3 cells, an adherent murine neuroblastoma cell line which expresses Ret and GFRα3, were plated at 2 x 10⁵ cells per well in 24-well plates in Dulbecco's modified eagle medium (DMEM), supplemented with 10 % fetal bovine serum, and cultured for 18 h at 37 °C and 5 % CO₂.

The cells were washed with PBS, and treated with serial dilutions of NBN in 0.25 mL of DMEM for 10 min at 37 °C and 5 % CO₂. Each sample was analyzed in duplicate. The cells were washed with 1 mL of PBS, and lysed for 1h at 4 °C with 0.30 mL of 10 mM Tris HCl, pH 8.0, 0.5 % Nonidet P40, 0.2 % sodium deoxycholate, 50 mM NaF, 0.1 mM Na₃ VO₄,1 mM phenylmethylsulfonyl fluoride with gently rocking the plates. The lysates were further agitated by repeated pipetting and 0.25 mL of sample was transferred to a 96- well ELISA plate that had been coated with 5 µg/mL of anti-Ret mAb (AA.GE7.3) in 50 mM carbonate buffer, pH 9.6 at 4°C for 18 h, and blocked at room temperature for one hour with block buffer (20 mM Tris HCl pH 7.5, 150 mM NaCl, 0.1% Tween-20 (TBST) containing 1 % normal mouse serum and 3 % bovine serum albumin).

After a 2 h incubation at room temperature, the wells were washed 6-times with TBST. Phosphorylated Ret was detected by incubating the wells at room temperature for 2 h with 2 :g/mL of horseradish peroxidase (HRP)-conjugated anti-phosphotyrosine 4GIO antibody in block buffer, washing 6-times with TBST, and measuring HRP activity at 450 nm with a colorometric detection reagent. The absorbance values from wells treated with lysate or with lysis buffer were measured and the background corrected signal was plotted as a function of the concentration of NBN present in the activation mixture. The potency of pegylated NBN in the KIRA ELISA was 10 nM, which is indistinguishable from that of the NBN NK starting material. There was no effect of two freeze-thaw cycles on potency and following this treatment there was no significant increase in the turbidity of the sample, indicating that the samples can be safely thawed for the study. In independent studies accessing the activity of product with three and four 10 kDa PEGs/molecule separately, it was determined that the adduct with three PEGs was fully active, while the four PEG product had reduced activity.

### Example 4. Pharmokinetic studies of internally pegylated rat neublastin in rats and mice

The pharmokinetic properties of various pegylated and non pegylated NBN products in rat and mouse models were examined.

The data revealed that pegylation of rat NBN NK with 3.3,10000 Da PEGs resulted in a significant effect on the half life and bioavailability of the neublastin. Following a 1 mg/kg IV administration in Sprague Dawley rats, peak levels of pegylated NBN of 3000 ng/mL were detected after 7 minutes, and levels of 700 ng/mL were detected after 24 h, 200 ng/mL after 48 h, and 100 ng/mL after 72 h. In contrast for non pegylated NBN following a 1 mg/kg IV administration, levels of 1500 ng/mL were detected after 7 minutes, but then the levels quickly dropped to 70 ng/mL after 3 h and were not detectable after 7 h. The effects of pegylation were even more pronounced in animals treated with pegylated NBN by subcutaneous administration.
Following a 1 mg/kg s.c. administration, circulating levels of pegylated NBN reached a maximum of 200 ng/mL after 24 h and remained at this level for the duration of the three day study. In contrast, no detectable NBN was observed at any time point following administration of unmodified NBN.

The analysis of the pegylated NBN samples are complicated by the presence of adducts containing 2, 3 and 4 PEGs per molecule, which each will display a different PK profile. In early PK studies, mice were used to facilitate screening through a variety of candidates and routes of administration. The mouse studies revealed dramatic differences in the bioavailability of the candidates. However, when the 3.3 10 K PEG adduct was evaluated in rats, it was found to be less bioavailable in rats than it was in mice. This difference in bioavailability was particularly pronounced following i.p. administration. Levels in mice reached 1600 ng/mL after 7 hr and remained at 400 ng/mL after 24 hr. In contrast, rat levels were constant at 100 ng/mL for 4-48 hr.

Both wild-type rat neublastin (wt-NBN) and neublastin with an Asn-to-Lys substitution at position 95 (NK-NBN) were refolded and purified to >95% for efficacy tests in the STZ diabetic rat neuropathy model. Wt-NBN was formulated to go directly into animal testing while NK-NBN was prepared for pegylation with 10 kDa PEG-SPA. To accomplish the refolding and purification goal, a refolding method utilizing size exclusion chromatography (SEC) was developed that permitted the renaturation of NBN from E.*coli* inclusion bodies in large quantities and at high concentrations. In addition to SEC, both Ni-NTA and CM silica column chromatography steps were employed to increase the final protein purity. The proteins were subjected to extensive characterization including analysis by SDS-PAGE, size exclusion chromatography, ESMS, assessment of activity by KIRA ELISA, and determination of endotoxin content. SDS-PAGE and SEC of the final protein products indicated a purity of greater than 95%. The endotoxin level of each product was < 0.2 EU/mg. The specific activity of both proteins in the KIRA ELISA is approximately 10 nM. Wt-NBN was formulated at 1.0 mg/ml and NK-NBN was formulated at 2.6 mg/ml in phosphate-buffered saline (PBS) pH6.5. wt-NBN was aliquoted into 15 ml tubes and stored frozen at -70°C while NK-NBN was subjected to pegylation prior to aliquoting and freezing.

### Example 5. Refolding of a wild-type neublastin and the N95K neublastin mutein

Both NBN forms were expressed in E. coli as a His-tagged fusion proteins with an enterokinase cleavage site immediately adjacent to the start of the mature 113 amino acid sequence. Bacteria expressing either Wt- (1.8 kg pellet) or NK-NBN (2.5 kg pellet) were subjected to lysis in 2 liters of PBS using a Gaulin press. Following centrifugation (10,000 rpm) to pellet the inclusion bodies, the supernatants from each preparation were discarded. The inclusion body pellets were washed two times with buffer (0.02M Tris-HCl pH 8.5, 0.5 mM EDTA) then washed two times with the same buffer containing Triton X-1 00 (2%, v/v) followed by two additional buffer washes without detergent. Both pellets were solubilized using 6M guanidine hydrochloride, 0.1M Tris pH 8.5, 0.1M DTT, and 1 mM EDTA. To aid in the solubilization process, each pellet was subjected to homogenization using a polytron homogenizer followed by overnight stirring at room temperature. The solubilized proteins were clarified by centrifugation prior to denaturing chromatography through Superdex 200 (5.5 liter column equilibrated with 0.05M glycine/H₃PO₄ pH 3.0 with 2M Guanidine-HCI) at 20 ml per minute.

Denatured NBN was identified by SDS-PAGE. Fractions containing either Wt-NBN or NK-NBN were pooled and concentrated to approximately 250 mL using an Amicon 2.5-liter stirred cell concentrator. After filtration to remove any precipitate, the concentrated protein was subjected to renaturing sizing chromatography through Superdex 200 equilibrated with 0.1 M Tris-HCl pH 7.8, 0.5M guanidine-HCl, 8.8 mM reduced glutathione and 0.22 mM oxidized glutathione. The column was developed using 0.5M guanidine-HCl at 20 mL per minute. Fractions containing renatured wt-NBN or NK-NBN were identified by SDS-PAGE, pooled, and stored at 4°C until needed for His tag removal.

### Concentration of NBN by Ni-NTA chromatography (TR5919-138).

Renatured NBN was stored at 4°C for at least 24 hours before proceeding with the purification to promote disulfide formation between the NBN monomers. During this time, a precipitate formed and was removed by filtration through a 0.2 µ PES filter unit. To decrease non-specific binding, the protein solution was brought to 20 mM imidazole prior to loading on a 100 ml Ni-NTA (Qiagen) column equilibrated with column buffer (0.5M guanidine and 20 mM imidazole) at 50 ml per minute. Following the protein application, the column was washed to baseline using the same buffer. NBN was eluted from the resin using approximately 300 mL of elution buffer containing 0.5M guanidine-HCl and 0.4M imidazole. After elution, NBN was dialyzed overnight (using 10 kDa dialysis tubing) at room temperature against ten volumes of 5 mM HCI. Dialysis promotes the hydrolysis of contaminating substances and decreases the guanidine-HCl and imidazole concentrations to 0.05M and 0.04M, respectively.

### Cleavage of the His tag by Lysyl Endopeptidase or Enterokinase.

The next day, any precipitate that formed during dialysis was removed by filtration. The protein sample was made 0.1 M NaCl by the addition of 5M stock for a final salt concentration including the remaining guanidine-HCl of approximately 150mM. This concentration was confirmed using a conductivity meter. Additionally, 1 M HEPFS pH 8 was added for a final concentration of 25mM. To cleave the tag, lysyl endopeptidase was added to wt-NBN and Enterokinase was added to NK-NBN, both at an approximately 1:300 ratio of protease to NBN. Enterokinase was used in place of lysyl endopeptidase for NK-NBN due to an additional protease cleavage site in the mutated protein at Lys95. The samples were stirred at room temperature for 2 hours and the digestions monitored by SDS-PAGE.

### His tag removal by Ni-NTA chromatography.

Protease-treated NBN was applied to a 100 mL Ni-NTA column equiliberated with 0.5M guanidine-HCl and 20 mM imidazole at 50 mL per minute. The column was washed to baseline with the same buffer. Any protein washing off the column was pooled with the flow-through protein containing NBN without the His tag.

### CM silica chromatography.

Following Ni-NTA chromatography, the protein was immediately subjected to further purification through CM silica resin. A 20 mL CM silica column equiliberated with loading buffer (5 mM phosphate pH 6.5, 150 mM NaCl) was loaded with NBN at 20 mL per minute. The column was washed with twenty column volumes of wash buffer (5 mM phosphate pH 6.5, 400 mM NaCl) and the protein step eluted with elution buffer containing 5 mM phosphate pH 6.5 but with 1 M NaCl. The eluted protein was dialyzed overnight against the phosphate alone to bring the salt concentration down to 100 mM for NK-NBN and 150 mM for wt-NBN. Both samples were filtered through a 0.2 µ PES filter unit, analyzed by SDS-PAGE, and stored at 4°C until needed for further characterizations and/or pegylation.

Wt-NBN and NK-NBN were subjected to UV spectrum analysis to assess their absorbance at 280. Using a micro quartz cuvette and blanking against buffer alone, 100 µl of either wt-NBN or NK-NBN was continuously scanned from 230 to 330 nm using a Beckman spectrophotometer. Based on this analysis, Wt-NBN was determined to be at a concentration of 1.1 mg/ml and NK-NBN at 2.6 mg/ml (A280 nm-E^{0.1%}=0.5 used for each protein). Less than 1% precipitated material was identified based on absorbance at 330 nm.

To assess the purity of both protein preparations, each sample (0.5 mg) was subjected to size exclusion chromatography through a 16/30 Superdex 75 column. The column was equiliberated with 5mM phosphate pH 6.5 containing 400 mM NaCl and developed with a 1.5 mL per minute flow rate. Based on the absorbance at 280 nm, both wt- and NK-NBN migrated as a single peak with an expected molecular weight (23-24 kDa), and they did not contain any significant protein contamination.

Both wt- and NK-NBN were reduced in 2.5 M guanidine-HCl, 60 mM Tris pH 8.0 and 16 mM DTT. The reduced samples were desalted over a short C₄ column and analyzed on-line by ESMS using a triple quadrupole instrument. The ESMS raw data were deconvoluted by the MaxEnt program to generate mass spectra. This procedure allows multiple charged signals to collapse into one peak that directly corresponds to the molecular mass in kilodaltons (kDa). The deconvoluted mass spectrum for wt-NBN showed the predominant species is 12046 kDa, which is in agreement with the predicted molecular weight of 12046.7 kDa for the 113 amino acid form of the protein. A minor component was also observed (12063 kDa) suggesting the presence of an oxidation product. Three peaks were identified in the NK-NBN sample. The major component demonstrated an apparent molecular mass of 11345 kDa in agreement with the predicted mass for the 106 amino acid form of the protein. The other two peaks had masses of 11362 and 12061 kDa, suggesting NK-NBN oxidation and the presence of the 113 amino acid form, respectively.

The presence of the 106 and 113 amino acid forms in the NK-NBN preparation is attributable to digestion with Enterokinase. This protease from Biozyme is a natural enzyme preparation purified from calf intestinal mucosa and is reported to contain a slight trypsin contamination (0.25 ng Trypsin per µg Enterokinase). Therefore, trypsin may be acting on NK-NBN on the carboxy terminal side of Arg7 to produce the predominant 106 amino acid form. On the other hand, Lysyl Endopeptidase used to cleave Wt-NBN is a single protease activity acting on the carboxy terminal side of the lysine residue contained within the His tag to produce the mature 113 amino acid NBN form. Both the 106 and 113 amino acid forms of NBN are equally active in all assays tested and behave similarly in guanidine-HCl stability tests.

NBN activity was determined by its ability to stimulate c-Ret phosphorylation in NB41A3-mRL3 cells using the KIRA ELISA described in Example 3. Phosphorylated Ret was detected by incubating (2 hours) the captured receptor with HRP-conjugated phosphotyrosine antibody (4G10; 0.2 µg per well). Following the incubation, the wells were washed six times with TBST, and the HRP activity detected at 450 nm with a colorimetric assay. The absorbance values from wells treated with lysate or with lysis buffer alone were measured, background corrected, and the data plotted as a function of the concentration of neublastin present in the activation mixture. The data demonstrate that the purified NBN resulted in the appearance of phosphorylated RET, indicating that the purified NBN was active in this assay.

### Example 6. Preparation of a serum aibumin-neublastin conjugate.

Wildtype rat neublastin at a concentration of 1 mg/ml in PBS was treated with 1 mM sulfo-SMCC (Pierce) and desalted to remove excess cross-linker. Since the wildtype NBN protein contains only a single amine at its N-terminus and no free sulfhydryl groups, reaction with SMCC was expected to result in site specific modification of the NBN with SMCC attached at its N-terminus.

60 µg of the NBN-SMCC conjugate was incubated with 120 µg of bovine serum albumin and analyzed for extent of cross-linking by SDS-PAGE. BSA contains a single free SH group and consequently reaction with the NBN-SMCC conjugate is expected to result in modification at this site through the maleimide on the SMCC. Under these conditions, two additional bands of higher molecular weight were observed, which are consistent in mass with modification of the NBN with a single BSA moiety and with two BSA molecules since each NBN molecule contains two N-termini that can undergo reaction, and consequently are in agreement with this notion. Concurrent with the formation of these bands, was a decrease in the intensity of the NBN-SMCC and BSA bands. Based on the intensity of the remaining NBN band the reaction appeared to have gone to 70-80% completion.

The monosubstituted product was purified from the reaction mixture by subjecting the material to cation exchange chromatography and size exclusion chromatography on a Superdex 200 column (Pharmacia) essentially as described for pegylation studies discussed above. Column fractions from the gel filtration run were analyzed by SDS-PAGE and those containing the monosubstituted product were analyzed for protein content by absorbance at 280 nm. Since the mass of BSA is approximately twice that of neublastin, the apparent concentration was divided by a factor of 3 to give the NBN equivalent. This fraction was subjected this to analysis for function in the KIRA ELISA. IC50 values for both the wt- and BSA-conjugated NBN were 3-6 nM, indicating that conjugation to the BSA had not compromised function.

While these preliminary studies were generated with BSA, the corresponding serum albumin proteins from rats and humans also contain a free SH. Consequently a similar approach can be applied to generate a rat serum albumin-rat NBN conjugate for performing PK and efficacy studies in rats and human serum albumin-human NBN for performing clinical trials. Similarly SMCC can be substituted with any of a number of cross-linkers that contain an amino reactive group on one side and a thiol reactive group on the other side. Examples of amine reactive cross-linkers that insert a thiol reactive-maleimide are AMAS, BMPS, MBS, EMCS, SMPB, SMPH, KMUS, or GMBS, that insert a thiol reactive-haloacetate group are SBAP, SIA, SIAB and that provide a protected or non protected thiol for reaction with sulfhydryl groups to product a reducible linkage are SPDP, SMPT, SATA, or SATP all of which are available from Pierce. Such cross linkers are merely exemplary and many alternative strategies are anticipated for linking the N-terminus of NBN with serum albumin. A skilled artisan also could generate conjugates to serum albumin that are not targeted at the N-terminus of NBN or at the thiol moiety on serum albumin. NBN-serum albumin fusions created using genetic engineering where NBN is fused to the serum albumin gene at its N-terminus, C-terminus, or at both ends, are also expected to be functional.

This method can be extended through routine adaptations to any NBN-serum albumin conjugate that would result in a product with a prolonged half-life in animals and consequently in humans.

## Claims

1. A variant neublastin polypeptide comprising an amino acid sequence at least 70% identical to amino acids 8-113 of SEQ ID NO:1,
provided that the variant neublastin polypeptide includes one or more of the amino acid substitutions selected from the group consisting of:
an amino acid other than arginine at position 14 in the amino acid sequence of said variant polypeptide,
an amino acid other than arginine at position 39 in the amino acid sequence of said variant polypeptide,
an amino acid other than arginine at position 68 of said variant polypeptide, and
an amino acid other than asparagine at position 95 of said variant polypeptide,
wherein the positions of said amino acids are numbered in accordance with the polypeptide sequence of SEQ ID NO:1.

2. The variant neublastin polypeptide of claim 1, wherein said variant neublastin polypeptide further comprises amino acids 1-7 of SEQ ID NO:1.

3. The variant neublastin polypeptide of claim 1, wherein said variant neublastin polypeptide, when dimerized, binds GFRα3.

4. The variant neublastin polypeptide of claim 1, wherein said variant neublastin polypeptide, when dimerized, stimulates tyrosine phosphorylation of a RET polypeptide.

5. The variant neublastin polypeptide of claim 1, wherein said variant neublastin polypeptide, when dimerized, enhances neuron survival.

6. The variant neublastin polypeptide of claim 1, wherein said variant neublastin polypeptide, when dimerized, enhances sensory neuron survival.

7. The variant neublastin polypeptide of claim 1, wherein said variant neublastin polypeptide, when dimerized, normalizes pathological changes of a neuron.

8. The variant neublastin polypeptide of claim 1, wherein said variant neublastin polypeptide, when dimerized, normalizes pathological changes of a sensory neuron.

9. The variant neublastin polypeptide of claim 1, wherein said variant neublastin polypeptide, when dimerized, enhances survival of an autonomic neuron.

10. The variant neublastin polypeptide of claim 1, wherein said variant neublastin polypeptide, when dimerized, enhances survival of a dopaminergic neuron.

11. The variant neublastin polypeptide of claim 1, wherein said variant polypeptide includes two or more of the amino acid substitutions selected from the group consisting of
an amino acid other than arginine at position 14 in the amino acid sequence of said variant polypeptide,
an amino acid other than arginine at position 39 in the amino acid sequence of said variant polypeptide,
an amino acid other than arginine at position 68 of said variant polypeptide, and
an amino acid other than asparagine at position 95 of said variant polypeptide.

12. The variant neublastin polypeptide of claim 1, wherein said variant polypeptide includes three or more of the amino acid substitutions selected from the group consisting of
an amino acid other than arginine at position 14 in the amino acid sequence of said variant polypeptide,
an amino acid other than arginine at position 39 in the amino acid sequence of said variant polypeptide,
an amino acid other than arginine at position 68 of said variant polypeptide, and
an amino acid other than asparagine at position 95 of said variant polypeptide.

13. The variant neublastin polypeptide of claim 1, wherein said variant polypeptide includes
an amino acid other than arginine at position 14 in the amino acid sequence of said variant polypeptide,
an amino acid other than arginine at position 39 in the amino acid sequence of said variant polypeptide,
an amino acid other than arginine at position 68 of said variant polypeptide, and
an amino acid other than asparagine at position 95 of said variant polypeptide.

14. The variant neublastin polypeptide of claim 1, wherein the amino acid at position 95 of said variant neublastin polypeptide is other than asparagine.

15. The variant neublastin polypeptide of claim 14, wherein the amino acid at position 95 of said variant neublastin polypeptide is lysine.

16. The variant neublastin polypeptide of claim 14, wherein said variant neublastin polypeptide further comprises a substitution selected from the group consisting of
an amino acid other than arginine at position 14;
an amino acid other than arginine at position 39, and
an amino acid other than arginine at position 68.

17. The variant neublastin polypeptide of claim 16, wherein said substitution at position 14, 39, or 68 is lysine.

18. The variant neublastin polypeptide of claim 14, wherein said variant neublastin polypeptide further comprises two substitutions selected from the group consisting of
an amino acid other than arginine at position 14;
an amino acid other than arginine at position 39, and
an amino acid other than arginine at position 68.

19. The variant neublastin polypeptide of claim 18 wherein said two substitutions are lysine.

20. The variant neublastin polypeptide of claim 14, wherein said variant neublastin polypeptide further comprises three substitutions as follows:
an amino acid other than arginine at position 14;
an amino acid other than arginine at position 39, and
an amino acid other than arginine at position 68.

21. The variant neublastin polypeptide of claim 20, wherein said three substitutions are lysine.

22. The variant neublastin polypeptide of claim 15, wherein amino acids 8-94 and 96-113 of said variant neublastin polypeptide are at least 90% identical to amino acids 8-94 and 96-113 of SEQ ID NO:1.

23. The variant neublastin polypeptide of claim 14, wherein amino acids 8-94 and 96-113 of said variant neublastin polypeptide are at least 95% identical to amino acids 8-94 and 96-113 of SEQ ID NO:1.

24. The variant neublastin polypeptide of claim 22, wherein the amino acid sequence of said variant neublastin polypeptide includes the amino acid sequence of a naturally occurring rat, human or mouse neublastin polypeptide at amino acids 8-94 and 96-113 of said variant neublastin polypeptide.

25. The variant neublastin polypeptide of claim 23, wherein amino acids 8-94 and 96-113 of said variant neublastin polypeptide are selected from the group consisting of the amino acid sequence of amino acids 8-94 and 96-113 of SEQ ID NO:2, SEQ ID NO:3 and SEQ ID NO:4.

26. The variant neublastin polypeptide of claim 15, wherein the polypeptide comprises amino acids 8-113 of SEQ ID NO:2.

27. The variant neublastin polypeptide of claim 1, wherein said variant neublastin polypeptide is a fusion protein.

28. The variant neublastin polypeptide of claim 27, wherein said fusion protein comprises a human serum albumin sequence fused in frame to said variant neublastin polypeptide.

29. A fusion protein comprising a neublastin polypeptide operably linked to a human serum albumin protein.

30. A nucleic acid molecule encoding the variant neublastin polypeptide of claim 1.

31. A vector comprising the nucleic acid of claim 30.

32. The vector of claim 30, wherein said vector is an expression vector.

33. A cell comprising the vector of claim 32.

34. The cell of claim 33, wherein said cell is selected from the group consisting of a mammalian cell, fungal cell, yeast cell, insect cell, and bacterial cell.

35. The cell of claim 34, wherein said mammalian cell is a Chinese hamster ovary cell.

36. A method of making a variant neublastin polypeptide, the method comprising culturing the cell of claim 33 under conditions allowing for expression of a variant neublastin polypeptide.

37. The method of claim 36, further comprising recovering said variant neublastin polypeptide.

38. A variant neublastin polypeptide produced by the method of claim 36.

39. A nucleic acid molecule encoding the variant neublastin polypeptide of claim 28.

40. A vector comprising the nucleic acid of claim 39.

41. The vector of claim 40, wherein said vector is an expression vector.

42. A cell comprising the vector of claim 41.

43. The cell of claim 42, wherein said cell is selected from the group consisting of a mammalian cell, fungal cell, yeast cell, insect cell, and bacterial cell.

44. The cell of claim 43, wherein said mammalian cell is a Chinese hamster ovary cell.

45. A method of making a variant neublastin polypeptide, the method comprising culturing the cell of claim 42 under conditions allowing for expression of a variant neublastin polypeptide.

46. The method of claim 45, further comprising recovering said variant neublastin polypeptide.

47. A variant neublastin polypeptide produced by the method of claim 46.

48. A multimeric polypeptide composition comprising the variant neublastin polypeptide of claim 1.

49. The multimeric polypeptide composition of claim 48, wherein said multimeric polypeptide composition is a dimer.

50. The multimeric polypeptide composition of claim 49, wherein said multimeric polypeptide composition is a homodimer.

51. The multimeric polypeptide composition of claim 48, wherein said multimeric polypeptide composition is a heterodimer.

52. A composition comprising a neublastin polypeptide or variant neublastin polypeptide coupled to a non-naturally occurring polymer.

53. A composition according to claim 52 wherein said non-naturally occurring polymer is coupled to said polypeptide via a solvent-exposed amino acid of said polypeptide.

54. The composition of claim 52, wherein said composition comprises a variant neublastin polypeptide comprising an amino acid sequence at least 70% identical to amino acids 8-113 of SEQ ID NO:1,
provided that the variant neublastin polypeptide includes one or more of the amino acid substitutions selected from the group consisting of:
an amino acid other than arginine at position 14 in the amino acid sequence of said variant polypeptide,
an amino acid other than arginine at position 39 in the amino acid sequence of said variant polypeptide,
an amino acid other than arginine at position 68 of said variant polypeptide, and
an amino acid other than asparagine at position 95 of said variant polypeptide, wherein the positions of said amino acids are numbered in accordance with the polypeptide sequence of SEQ ID NO:1.

55. The composition of claim 52, wherein said polymer comprises a polyalkylene glycol moiety.

56. The composition of claim 55, wherein said polyalkylene glycol moiety is a polyethylene glycol moiety (PEG).

57. The composition of claim 55, wherein said polyalkylene glycol moiety is coupled to an amine group of said neublastin polypeptide or a lysine or other amine group of a variant neublastin polypeptide.

58. The composition of claim 57, wherein said polyalkylene glycol moiety is coupled by a N-hydroxylsuccinimide (NHS) active ester.

59. The composition of claim 58, wherein said active ester is selected from the group consisting of PEG succinimidyl succinate (SS-PEG), succinimidyl butyrate (SPB-PEG), and succinimidyl propionate (SPA-PEG).

60. The composition of claim 57, wherein said polyalkylene glycol moiety is selected from the group consisting of carboxymethyl-NHS, norleucine-NHS, SC-PEG, tresylate, aldehyde, epoxide, carbonylimidazole, and PNP carbonate

61. The composition of claim 55, wherein said polyalkylene glycol moiety is coupled to a cysteine group of said neublastin polypeptide or variant neublastin polypeptide.

62. The composition of claim 61, wherein said polyalkylene glycol moiety is coupled by way of a group selected from the group consisting of a maleimide group, a vinylsulfone group, a haloacetate group, and a thiol group.

63. The composition of claim 52, wherein said neublastin polypeptide or variant neublastin polypeptide is glycosylated.

64. The composition of claim 63, wherein said polymer is coupled to a carbohydrate moiety of said glycosylated neublastin polypeptide or variant neublastin polypeptide.

65. The composition of claim 64, wherein said polymer is coupled to said glycosylated neublastin polypeptide or variant neublastin polypeptide following oxidation of a hydrazole group or an amino group of said glycosylated neublastin polypeptide or variant neublastin polypeptide.

66. The composition of claim 52, wherein said neublastin polypeptide or variant neublastin polypeptide comprises one two, three, or four PEG moieties.

67. The composition of claim 52, wherein the neublastin polypeptide or variant neublastin polypeptide has a longer serum half-life relative to the half-life of said polypeptide or variant polypeptide in the absence of said polymer.

68. The composition of claim 52, wherein the neublastin polypeptide or variant neublastin polypeptide, when dimerized, has a physiological activity selected from the group consisting of: GFRα3 binding, RET activation, normalization of pathological changes of a neuron, and enhancing neuron survival.

69. The composition of claim 68, wherein said polymer is coupled to said polypeptide at an N terminus thereof.

70. The composition of claim 68, wherein said polymer is coupled to said polypeptide at a non-terminal amino acid thereof.

71. The composition of claim 68, wherein said polymer is coupled to a solvent exposed amino acid of the neublastin polypeptide or variant neublastin polypeptide.

72. The composition of claim 68, wherein said polymer is coupled to said neublastin polypeptide or variant neublastin polypeptide at a residue selected from the group consisting of: the amino terminus of said variant polypeptide, position 14 in the amino acid sequence of said neublastin polypeptide or variant neublastin polypeptide, position 39 in the amino acid sequence of said neublastin polypeptide or variant neublastin polypeptide, position 68 in the amino acid sequence of said neublastin polypeptide or variant neublastin polypeptide, and position 95 in the amino acid sequence of said neublastin polypeptide or variant polypeptide.

73. A pharmaceutical composition comprising a physiologically acceptable vehicle having dispersed therein, the composition of claim 68.

74. A stable, aqueously soluble conjugated neublastin polypeptide or variant neublastin polypeptide complex comprising a dimeric neublastin polypeptide or dimeric variant neublastin polypeptide coupled to a polyethylene glycol moiety, wherein the neublastin polypeptide or variant neublastin polypeptide is coupled to the polyethylene glycol moiety by a labile bond.

75. The complex of claim 74, wherein said labile bond is cleavable by biochemical hydrolysis, proteolysis, or sulfhydryl cleavage.

76. The complex of claim 75, wherein said labile bond is cleavable under in vivo conditions.

77. A method for making a modified neublastin polypeptide that, when dimerized, has prolonged activity in vitro or in vivo, relative to a wild-type neublastin polypeptide, when dimerized, the method comprising
providing a neublastin polypeptide or variant neublastin polypeptide; and
coupling said polypeptide or modified variant neublastin polypeptide to a non-naturally occurring polymer moiety, thereby forming a coupled polymer neublastin polypeptide composition.

78. A method of treating or preventing a nervous system disorder in a subject, the method comprising administering to a subject in need thereof a therapeutically effective amount of: a dimer comprising the polypeptide of claim 1, the composition of claim 52 comprising a dimer of said neublastin polypeptide or variant neublastin polypeptide conjugate, the pharmaceutical composition of claim 69, or the complex of claim 74.

79. The method of claim 78, wherein said nervous disorder is a peripheral nervous disorder.

80. The method of claim 78, wherein said disorder is a peripheral neuropathy.

81. Wherein said disorder is a neuropathic pain syndrome.

82. The method of claim 78, wherein said subject is a human.

83. The method of claim 78, wherein administration is systemic.

84. The method of claim 78, wherein administration is local.


SEQUENCE LISTING

<110> Biogen, Inc.
      Sah, Dinah W.Y.
      Pepinsky, Blake R
      Borjack-Sjodin, Paula Ann
      Miller, Stephan S
      Rossomando, Anthony

<120> Polymer Conjugates of Neublastin and Methods of Using
      Same

<130> 00689-506-061

<140> Not Yet Assigned
<141> 2002-01-23

<150> 60/266,071
<151> 2001-02-01

<160> 13

<170> PatentIn Ver. 2.1

<210> 1
<211> 111
<212> PRT
<213> Artificial Sequence

<220>
<223> Description of Artificial Sequence: Consensus
      Sequence

<220>
<221> VARIANT
<222> (3)
<223> Wherein Xaa is Gly or Thr

<220>
<221> VARIANT
<222> (4)
<223> Wherein Xaa is Pro or Arg

<220>
<221> VARIANT
<222> (5)
<223> Wherein Xaa is Gly or Ser

<220>
<221> VARIANT
<222> (10)
<223> Wherein Xaa is Ala or Thr

<220>
<221> VARIANT
<222> (11)
<223> Wherein Xaa is Ala or Thr

<220>
<221> VARIANT
<222> (12)
<223> Wherein Xaa is Gly or Asp

<220>
<221> VARIANT
<222> (26)
<223> Wherein Xaa is Arg or Ser

<220>
<221> VARIANT
<222> (33)
<223> Wherein Xaa is Arg or Ser

<220>
<221> VARIANT
<222> (38)
<223> Wherein Xaa is Val or Ile

<220>
<221> VARIANT
<222> (51)
<223> Wherein Xaa is Val or Ile

<220>
<221> VARIANT
<222> (53)
<223> Wherein Xaa is Pro or Gln

<220>
<221> VARIANT
<222> (69)
<223> Wherein Xaa is Pro or Ser

<220>
<221> VARIANT
<222> (76)
<223> Wherein Xaa is Val or Ile

<220>
<221> VARIANT
<222> (103)
<223> Wherein Xaa is Arg or His

<400> 1
Ala Gly Xaa Xaa Xaa Ser Arg Ala Arg Xaa Xaa Xaa Ala Arg Gly Cys
1               5                   10                  15

Arg Leu Arg Ser Gln Leu Val Pro Val Xaa Ala Leu Gly Leu Gly His
            20                  25                  30

Xaa Ser Asp Glu Leu Xaa Arg Arg Phe Cys Ser Gly Ser Cys Arg Arg
        35                  40                  45

Ala Arg Ser Xaa His Asp Leu Ser Leu Ala Ser Leu Leu Gly Ala Gly

    50                  55                  60

Ala Leu Arg Xaa Pro Pro Gly Ser Arg Pro Xaa Ser Gln Pro Cys Arg
65                  70                  75                  80

Pro Thr Arg Tyr Glu Ala Val Ser Phe Met Asp Val Asn Ser Thr Trp
                85                  90                  95

Arg Thr Val Asp Xaa Leu Ser Ala Thr Ala Cys Gly Cys Leu Gly
            100                 105                 110

<210> 2
<211> 113
<212> PRT
<213> Homo sapiens

<400> 2
Ala Gly Gly Pro Gly Ser Arg Ala Arg Ala Ala Gly Ala Arg Gly Cys
1               5                   10                  15

Arg Leu Arg Ser Gln Leu Val Pro Val Arg Ala Leu Gly Leu Gly His
            20                  25                  30

Arg Ser Asp Glu Leu Val Arg Phe Arg Phe Cys Ser Gly Ser Cys Arg
        35                  40                  45

Arg Ala Arg Ser Pro His Asp Leu Ser Leu Ala Ser Leu Leu Gly Ala
    50                  55                  60

Gly Ala Leu Arg Pro Pro Pro Gly Ser Arg Pro Val Ser Gln Pro Cys
65                  70                  75                  80

Cys Arg Pro Thr Arg Tyr Glu Ala Val Ser Phe Met Asp Val Asn Ser
                85                  90                  95

Thr Trp Arg Thr Val Asp Arg Leu Ser Ala Thr Ala Cys Gly Cys Leu
            100                 105                 110

Gly

<210> 3
<211> 113
<212> PRT
<213> Mus musculus

<400> 3
Ala Gly Thr Arg Ser Ser Arg Ala Arg Thr Thr Asp Ala Arg Gly Cys
1               5                   10                  15

Arg Leu Arg Ser Gln Leu Val Pro Val Ser Ala Leu Gly Leu Gly His
            20                  25                  30

Ser Ser Asp Glu Leu Ile Arg Phe Arg Phe Cys Ser Gly Ser Cys Arg
        35                  40                  45

Arg Ala Arg Ser Gln His Asp Leu Ser Leu Ala Ser Leu Leu Gly Ala
    50                  55                  60

Gly Ala Leu Arg Ser Pro Pro Gly Ser Arg Pro Ile Ser Gln Pro Cys
65                  70                  75                  80

Cys Arg Pro Thr Arg Tyr Glu Ala Val Ser Phe Met Asp Val Asn Ser
                85                  90                  95

Thr Trp Arg Thr Val Asp His Leu Ser Ala Thr Ala Cys Gly Cys Leu
            100                 105                 110

Gly

<210> 4
<211> 113
<212> PRT
<213> Rattus norvegicus

<400> 4
Ala Gly Thr Arg Ser Ser Arg Ala Arg Ala Thr Asp Ala Arg Gly Cys
1               5                   10                  15

Arg Leu Arg Ser Gln Leu Val Pro Val Ser Ala Leu Gly Leu Gly His
            20                  25                  30

Ser Ser Asp Glu Leu Ile Arg Phe Arg Phe Cys Ser Gly Ser Cys Arg
        35                  40                  45

Arg Ala Arg Ser Pro His Asp Leu Ser Leu Ala Ser Leu Leu Gly Ala
    50                  55                  60

Gly Ala Leu Arg Ser Pro Pro Gly Ser Arg Pro Ile Ser Gln Pro Cys
65                  70                  75                  80

Cys Arg Pro Thr Arg Tyr Glu Ala Val Ser Phe Met Asp Val Asn Ser
                85                  90                  95

Thr Trp Arg Thr Val Asp His Leu Ser Ala Thr Ala Cys Gly Cys Leu
            100                 105                 110

Gly

<210> 5
<211> 675
<212> DNA
<213> Rattus norvegicus

<400> 5
atggaactgg gacttggaga gcctactgca ttgtcccact gcctccggcc taggtggcaa 60
ccagccttgt ggccaaccct agctgctcta gccctgctga gcagcgtcac agaagcttcc 120
ctggacccaa tgtcccgcag ccccgcctct cgcgatgttc cctcgccggt cctggcgccc 180

ccaacagact acctacctgg gggacacacc gcacatctgt gcagcgaaag agccctgcga 240
ccaccgccgc agtctcctca gcccgcaccc ccaccaccgg gtcccgcgct ccagtctcct 300
cccgctgcgc tccgcggggc acgcgcggcg cgtgcaggaa cccggagcag ccgcgcacgg 360
gctacagatg cgcgcggctg ccgcctgcgc tcacagctgg tgccggtgag cgctctcggc 420
ctgggccaca gctccgacga gctgatacgt ttccgcttct gcagcggttc gtgccgccga 480
gcacgctccc cgcacgatct cagcctggcc agcctgctgg gcgccggggc cctgcggtct 540
cctcccgggt cccggccgat cagccagccc tgttgccggc ccactcgcta tgaggcagtc 600
tccttcatgg acgtgaacag cacctggaga accgtggacc atctctccgc caccgcctgc 660
ggctgtctgg gctga 675

<210> 6
<211> 57
<212> DNA
<213> Artificial Sequence

<220>
<223> Description of Artificial Sequence: KD2-310
      Oligonucleotide

<400> 6
gtatctttca tggacgtaaa gtctacatgg agaaccgtag atcatctatc tgcaacc 57

<210> 7
<211> 57
<212> DNA
<213> Artificial Sequence

<220>
<223> Description of Artificial Sequence: KD3-211
      Oligonucleotide

<400> 7
ggttgcagat agatgatcta cggttctcca tgtagacttt acgtccatga aagatac 57

<210> 8
<211> 50
<212> DNA
<213> Artificial Sequence

<220>
<223> Description of Artificial Sequence: KD3-254
      Oligonucleotide

<400> 8
gctggaactc gcagctctcg tgctcgtgca acggatgcaa aaggctgtcg 50

<210> 9
<211> 50
<212> DNA
<213> Artificial Sequence

<220>
<223> Description of Artificial Sequence: KD2-255

Oligonucleotide

<400> 9
cgacagcctt ttgcatccgt tgcacgagca cgagagctgc gagttccagc 50

<210> 10
<211> 37
<212> DNA
<213> Artificial Sequence

<220>
<223> Description of Artificial Sequence: KD3-258
Oligonucleotide

<400> 10
ggagccggag cactaaaatc tccccgggat ctagacc 37

<210> 11
<211> 38
<212> DNA
<213> Artificial Sequence

<220>
<223> Description of Artificial Sequence: KD3-259
Oligonucleotide

<400> 11
ggtctagatc ccgggggaga ttttagtgct ccggctcc 38

<210> 12
<211> 32
<212> DNA
<213> Artificial Sequence

<220>
<223> Description of Artificial Sequence: KD3-256
Oligonucleotide

<400> 12
gacgaattaa ttaagtttcg tttttgttca gg 32

<210> 13
<211> 32
<212> DNA
<213> Artificial Sequence

<220>
<223> Description of Artificial Sequence: KD3-257
Oligonucleotide

<400> 13
cctgaacaaa aacgaaactt aattaattcg tc 32